# EUROPEAN PATENT SPECIFICATION

(11) **EP 3 295 981 B1**
(45) Date of publication and mention of the grant of the patent: **15.07.2020**
(21) Application number: 17191756.0
(22) Date of filing: 19.09.2017
(51) Int. Cl.: A61M 5/315, A61M 5/142, A61M 5/31

(54) **ROTATION RESISTANT FRICTION ADAPTER FOR PLUNGER DRIVER OF DRUG DELIVERY DEVICE**
ROTATION WIDERSTEHENDER REIBADAPTER FÜR DEN KOLBENTREIBER EINER ARZNEIMITTELAUSGABEVORRICHTUNG
ADAPTATEUR DE FRICTION ANTI ROTATION D'ENTRAINEMENT DE PISTON DANS UN DISPOSITIF D'ADMINISTRATION DE MÉDICAMENT

(30) Priority: 19.09.2016 US 201615269248
(43) Date of publication of application: 21.03.2018
(73) Proprietor: Medimop Medical Projects Ltd., 43665 Ra'anana (IL)
(72) Inventor: CABIRI, Oz, 71799 Macabim-Reut (IL); HEZKIAHU, Ran, 4668346 Herzliya (IL)
(74) Representative: Lippert Stachow Patentanwälte Rechtsanwälte

(56) References cited:
- US-A1- 2009 093 792

## Description

### BACKGROUND OF THE INVENTION

The present invention, in some embodiments thereof, relates to an anti-rotational friction fitting, more particularly, but not exclusively, to a fitting for preventing rotation of a plunger driver of a drug delivery device.

U.S. Patent No. 7967795 to the present inventor (Oz Cabiri) discloses "a cartridge interface assembly including a driving plunger including an outer shaft, and a driver including an inner shaft, the inner shaft mating with an intermediate shaft, the intermediate shaft mating with the outer shaft, so that the shafts are movable telescopically with respect to one another, wherein rotation of the driver causes the driving plunger to advance in a direction away from the driver". US 2009/093792 A1 discloses a device according to the preamble of claim 1.

### BRIEF SUMMARY OF THE INVENTION

The invention is defined by the subject-matter of the independent claims. According to an aspect of some embodiments of the invention, there is provided an assembly for driving a plunger and in a drug reservoir, the plunger dividing between a drug in a distal portion of the reservoir and a proximal opening of the reservoir; the assembly comprising: a telescoping shaft that extends by rotation of a driving element in a first rotational direction with respect to a distal shaft; a distal end of the distal shaft configured to engage the plunger; and a radial element extending from the distal shaft to contact an interior side wall of the reservoir, the element configured and oriented to wedge between the interior wall and the distal shaft when the distal shaft is rotated in the first rotational direction.

According to some embodiments of the invention, the element includes a sprag having a long axis and wherein the long axis intersects the interior side wall.

According to some embodiments of the invention, the long axis intersects the interior side wall at an angle between 91 and 120 degrees from the first rotational direction.

According to some embodiments of the invention, the element includes a virtual beam having a preferred bending direction opposite the first rotational direction.

According to some embodiments of the invention, the assembly further comprises: a plunger interface configured to engage the plunger; the plunger interface irrotationally interconnecting the distal shaft and the plunger when the plunger interface is engaged to the plunger.

According to some embodiments of the invention, the assembly further comprises: a vent for releasing gas from a space between the element and the plunger.

According to some embodiments of the invention, the radial element is located proximal to the distal end of the distal shaft.

According to some embodiments of the invention, the wedging is longitudinal such that moving the distal shaft distally with respect to the reservoir reduces a normal force between the radial element and the interior wall.

According to some embodiments of the invention, a resistance due to friction between the radial element and the interior wall to movement of the distal shaft in the first rotational direction is higher resistance due to friction between the radial element and the interior wall to movement of the distal shaft in a direction opposite the first rotational direction.

According to some embodiments of the invention, the assembly further comprises: a second friction element extending from a drug delivery device to contact an exterior side wall of the reservoir when the reservoir is inserted into the drug delivery device, the second friction element is configured and oriented to wedge between the exterior wall and the reservoir when the reservoir is rotated in a second rotational direction.

According to some embodiments of the invention, the second rotational direction is opposite the first rotational direction.

According to an aspect of some embodiments of the invention, there is provided a method of advancing a plunger in a drug reservoir comprising: providing a telescoping assembly that extends by rotating a driver in a first direction with respect to a pushing shaft mated to the driver, the pushing shaft including a friction element in contact with a wall of the reservoir; rotating the driver in the first direction; wedging the friction element between the pushing shaft and the wall as result of the rotating; inhibiting rotation of the first direction as a result of the wedging; and unwedging the friction element to facilitating advancing of the pushing shaft.

According to some embodiments of the invention, the wedging is substantially the only process impeding rotation of the pushing shaft with respect to the reservoir.

According to some embodiments of the invention, wedging include increasing normal force.

According to some embodiments of the invention, the method further comprises: reducing a normal force between the friction element and the wall of the reservoir when the pushing shaft moves in a distal direction.

According to some embodiments of the invention, the drug reservoir includes a plunger, the method further comprising: reducing a normal force between the friction element and the wall of the reservoir when the pushing shaft pushes distally against the plunger.

According to an aspect of some embodiments of the invention, there is provided a drug delivery device comprising: a housing including a channel; a drug reservoir fitting into the channel; and a friction element extending from a housing to the channel to contact an exterior side wall of the reservoir when the reservoir is inserted into the channel, the element configured and oriented to wedge between the exterior wall and the reservoir when the reservoir is rotated in a first rotational direction.

According to some embodiments of the invention, the assembly further comprises: a telescoping shaft that extends by rotation of a driving element in a first rotational direction with respect to a shaft; and a second friction element inhibiting rotation of the shaft with respect to the reservoir.

According to some embodiments of the invention, the channel is cylindrical and includes an opening at one end and wherein the reservoir slides longitudinally through the opening into the channel and wherein the friction element has increased friction resistance to movement in the first rotational direction than to movement in the longitudinal direction.

According to an aspect of some embodiments of the invention, there is provided a method of distributing a drug comprising: supplying a reservoir containing the drug and a distribution device including a channel and a friction element; inserting the reservoir into longitudinally into the channel; and wedging the reservoir against the friction element by rotating the reservoir with respect to the device.

According to an aspect of some embodiments of the invention, there is provided an assembly for driving a plunger in a drug reservoir, the plunger dividing between a drug in a distal portion of the reservoir and a proximal opening of the reservoir; the assembly comprising: a telescoping shaft that extends by rotation of a driving element in a first rotational direction with respect to a distal shaft; a distal end of the distal shaft configured to engage the plunger; a radial element extending from the distal shaft to contact an interior side wall of the reservoir, and a vent allowing gas to pass from an interior of the reservoir between the plunger and the radial element toward the proximal opening of the reservoir, the vent formed in at least one component selected from the group consisting of in the radial element, between the radial element and the side wall of the reservoir, in the distal shaft and between the distal shaft and the radial element.

According to some embodiments of the invention, the assembly includes at least two vents in a least two components and wherein the at least two vents are aligned such that gas passing through a first vent in a first of the two components is channeled to a second vent is a second of the components.

According to some embodiments of the invention, the assembly further comprises a user indicator visible from outside the drug reservoir and wherein the assembly is configured to move the indicator along a trajectory and wherein a position of the indicator indicates a status of the assembly.

According to some embodiments of the invention, the indicator is not visible from outside the reservoir over at least a portion of the trajectory.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flow chart illustration of a method of facilitating movement of a plunger driver in accordance with embodiments of the current invention;
FIG. 2 is a flow chart illustration of a method of driving a plunger in accordance with embodiments of the current invention;
FIG. 3A is a block diagram illustration of an assembly for driving a plunger in accordance with embodiments of the current invention;
FIGs. 3B-3C are schematic diagrams of a sprag element resisting movement in a hindered direction and allowing movement in a direction other than the hindered direction in accordance with embodiments of the current invention;
FIGs. 4A, 4B, 4C, 4D, 4E and 4F are drawings of a plunger driving assembly and drug reservoir with a threaded plunger interface in accordance with embodiments of the current invention;
FIG. 5 is a perspective driving of a plunger pushing rod and friction element for facilitating forward linear motion in accordance with embodiments of the current invention;
FIGs. 6A-6B are cross sectional views of a plunger pushing rod including flexible beam anisotropic friction elements in accordance with embodiments of the current invention;
FIGs. 7A-7B are side views of a plunger pushing assembly with reduced friction when engaged to a plunger in accordance with embodiments of the current invention;
FIG. 8 is a perspective view of a rod including a sprag friction elements and a ram plunger interface in accordance with embodiments of the current invention;
FIG. 9 is a perspective view of a rod including virtual sprag friction elements in accordance with embodiments of the current invention;
FIG. 10A is a perspective view of a cartridge including a wheeled friction element in accordance with an embodiment of the current invention;
FIG. 10B is a perspective view of a wheeled friction element with a shaft mount in accordance with an embodiment of the current invention;
FIG. 10C is a perspective view of a wheeled friction element without a shaft mount in accordance with an embodiment of the current invention;
FIG. 11 is a perspective view of a cartridge held by an external friction annular element in accordance with an embodiment of the current invention;
FIG. 12A is a perspective view of a cartridge including an external semi annular friction element in accordance with an embodiment of the current invention;
FIG. 12B is a perspective view of a cartridge including an external semi annular friction element installed into a drug delivery device in accordance with an embodiment of the current invention;
FIG. 13A is a flow chart illustration of a method of retaining a plunger driver to a reservoir in accordance with an embodiment of the current invention;
FIG. 13B is a flow chart illustration of assembly and/or use of a drug delivery device and/or a drug cartridge in accordance with an embodiment of the current invention;
FIG. 14A illustrates a medicine cartridge in accordance with an embodiment of the current invention;
FIG 14B illustrates a cartridge coupled to a delivery device in accordance with an embodiment of the current invention; and
FIG. 15 illustrates a connection between a friction pad and a plunger driver in accordance with an embodiment of the current invention.

### DETAILED DESCRIPTION OF THE INVENTION

### OVERVIEW

The present invention, in some embodiments thereof, relates to an anti-rotational friction fitting, more particularly, but not exclusively, to a fitting for preventing rotation of a plunger driver and/or a reservoir of a drug delivery device.

An aspect of some embodiments of the present invention relates to a variable friction element facilitating forward movement of a shaft inside a drug reservoir. For example, forward motion of the pushing shaft may be driven by rotation of a driver element with respect to the pushing shaft. The friction element may facilitating the relative rotation for example by inhibiting the pushing shaft from rotating along with the driving element and/or by inhibiting rotation of the reservoir. Alternatively or additionally, the friction element may be designed to reduce friction to forward movement of the pushing shaft and/or reservoir (facilitating movement in the forward, preferred direction).

In some embodiments, the friction element may facilitate and/or speed up forward movement of the pushing shaft and/or plunger interface prior to contact with the plunger. Optionally, when the pushing shaft is pushing the plunger, resistance to forward movement due to the friction element may be reduced and/or may self-adjust to minimal and/or may be negligible.

As used herein the term plunger means a piston element. For example, a plunger may include a plunger shaft and/or a plunger head and/or a plunger seal and/or a mating element. As used herein, the term/phrase plunger seal means a piston element that impedes fluid flow. Optionally plunger seal includes and/or is integral to a piston head. For example, the seal may have the form of a stopper and/or a gasket. In some embodiments, the term plunger may refer to the plunger seal alone. For example, the plunger may include a single sealing element that is pushed by a driver. Optionally, the driver may include a pushing shaft.

In some embodiments, the friction element is configured to wedge and/or increase a normal force between an outer wall of the shaft and an inner wall of the reservoir when the shaft moves in an inhibited direction with respect to the reservoir. In some embodiments, the friction element is configured to wedge and/or increase a normal force between an outer wall of the reservoir and a drug delivery device when the reservoir moves in an inhibited direction with respect to the delivery device. Alternatively or additionally, the friction element is configured to become dislodged and/or reduce a normal force between an outer wall of the shaft and in inner wall of the reservoir when said shaft moves in a preferred direction with respect to the reservoir. In some embodiments, the preferred direction of movement is in a direction that causes the rod to approach the plunger and/or discharge the drug, for example advancement, for example in the distal direction.

In some embodiments, the friction element may include a sprag and/or a beam. For example, a beam may have a preferential mode of bending that causes anisotropic friction. Optionally the sprag and/or beam may include a separate element. Alternatively or additionally, the sprag and/or beam may be integral to another element and/or other elements. As used herein the term virtual element (for example virtual sprag and/or virtual beam) means an element that is intrinsic to another element. For example, a virtual sprag and/or beam may be a region of a radial element (for example the radial element may include a disk) having decreased compressibility in comparison to a surrounding region. For example, a virtual sprag and/or beam may include a rib on a radial element.

In some embodiments, a sprag may be an element that is propped between two surfaces at an angle such that a movement of one of the surfaces in first direction compresses the sprag and/or wedges the sprag between the two surfaces thereby increasing a normal force and/or a resistance to the movement. Alternatively or additionally, movement of the surface in a direction different from the first direction may reduce a normal force and/or decrease resistance to the movement and/or reducing resistance to another movement. A virtual and/or a combined sprag may include a portion of an element having properties that cause variable friction between two surface such that a movement in first direction compresses the sprag increasing resistance to the movement and/or a movement in a direction different from the first direction releases a force on the sprag decreasing resistance to the opposite movement and/or to any movement. For example, frictional resistance to movement in the inhibited direction may range between 2x to 4x as large and/or between 4x to 10x and/or between 10x to 100x or greater than 100x as great as frictional resistance to movement in an another direction (for example an opposite direction from the first direction) and/or as frictional resistance to movement in the preferred direction. For example, movement in the preferred direction may reduce frictional resistance to movement by 2x to 4x and/or by 4x to 10x and/or by 10x to 100x or greater than 100x. The combination of increasing frictional resistance to movement in a non-preferred direction while reducing frictional resistance to movement in a preferred direction may reduce and/or minimize friction resistance to movement in the preferred direction while increasing and/or maximizing resistance to other movements.

In some embodiments, a friction fitting and/or a plunger interface may include an air vent. The air vent may prevent buildup of an air pocket between the plunger interface and the plunger seal as the pushing shaft and/or plunger interface approaches the plunger seal.

In some embodiments, the friction element of the present invention may work with various forms of medicine reservoirs and/or plungers. For example, the invention may be configured for a standard syringe, cartridge, reservoir and/or plunger. For example, the cavity of the reservoir may be cylindrical. For example, the cavity of the reservoir may have smooth walls.

In some embodiments, a friction element may also include a user indicator. For example, a friction element may be colored and/or easily identified and/or be illuminated. Optionally, the position of the friction element is visible the user, for example through a window in the device and/or through a side wall of a reservoir. The positional and/or illumination of the friction element optionally indicates a state of the device.

In some embodiments, movement of the friction element may indicate that the device is working. For example, movement of the friction element may reveal that the device is active before the anti friction element reaches the plunger seal.

In some embodiments, the friction element and/or a base (e.g. a support element and/or connector) of the friction element may change appearance depending on a state of the device. For example, the appearance may change when the friction element reaches a plunger seal.

In some embodiments, the indicator will be visible to the user over its entire trajectory. Alternatively or additionally, the indicator may be visible over only a part of its trajectory. For example, a widow of the drug discharge device may only expose the portion of the trajectory. Alternatively or additionally, a wall of a drug reservoir may be transparent over part of the trajectory and/or not transparent over another part of the trajectory. For example, the window and/or reservoir may only expose the portion of the trajectory. For example, the driver, base and/or friction element may become exposed when they contact the plunger seal. The exposure optionally indicates that discharge has started. Alternatively or additionally, the driver, base and/or friction element may be exposed at their initial position and/or may be concealed (for example by an opaque portion of the window and/or housing of the device) just after that point, for example right after the driver starts moving towards the plunger seal in may be exposed or concealed. For example, the change in appearance of the friction element may be an indicator that the system has started working and/or is working properly.

In some embodiments, at the end of their trajectory the plunger, driver, base, friction element and/or plunger seal may be concealed. For example, the disappearance of the element may be a sign that discharge was successfully completed. For example, a marked portion of the driver may be covered by a plunger seal when the driver contacts the plunger seal.

In some embodiments, the window and/or reservoir wall may include a tinted pane over one or more portions of the trajectory. For example, the tinting may cause a change in appearance of the driver, base, friction element and/or plunger seal when they reach a certain point on their trajectory. For example, the change in color may indicate a change is status of the discharge device. In some embodiment, the indicator may depend on a part supplied by a device manufacturer, for example the friction element and/or a driver element and/or a part of the delivery device (for example to indicate a status that is inherent to the device. Alternatively or additionally, the indicator may be dependent on the drug packer, for example a plunger seal (for example in cases where the indicated status is related to the particular drug.

An aspect of some embodiments of the present invention relates to an element that produces high friction when the pushing shaft is moving with minimal external resistance and/or produces reduced friction when the pushing shaft is pushing against a plunger of the reservoir. For example, the plunger interface may be configured to cause the friction element to fold and/or bend away from the walls of the reservoir and/or reduce friction when the interface engages the plunger.

In some embodiments, the plunger interface may include a standard connector. For example, the plunger interface may include a screw that screws into the plunger. The interface may transfer linear force and/or rotational torque between the pushing shaft and the plunger. Alternatively or additionally, the interface may transfer linear force in one or more directions while transferring limited and/or negligible torque between the pushing shaft and the plunger. For example, a plunger interface may include a screw thread. Until the screw interface reaches the plunger, the friction element may inhibit rotation and/or facilitate linear movement of the pushing shaft. As the screw element engages between the pushing shaft and the plunger, resistance is optionally added against forward motion of the pushing shaft and/or the friction inhibiting rotation of the pushing shaft is optionally reduced. Optionally, the interface and/or the pushing shaft will rotate, engaging the screw threads. At some point of engagement of the screw threads (for example when they are fully screwed together and/or fully engaged), the resistance of the plunger to rotation will optionally stop and/or reduce rotation of the pushing shaft and/or facilitate further linear advance of the plunger and/or pushing shaft. Alternatively or additionally, a plunger interface may include a locking element, for example a plug and socket that transfers torque and/or linear force between the pushing shaft and the plunger. Alternatively or additionally, the plunger may include a ram interface with pushes the plunger without transferring significant rotational torque.

In some embodiments, a friction element may be adjusted according the drive system, injection schedule and/or load on the plunger. For example, for a high viscosity payload where the load on the injector is high, the friction element may be designed with lower friction resistance to forward movement. For example, where injection is fast (e.g. for a for a high pitch driving screw) the friction element may be designed with lower friction resistance to forward movement. For example, for a reservoir having approximately 100 g*cm of torque generated pushing the drug during delivery, a friction element may have a torque resistance in an non-preferred direction of approximately 90 g*cm and/or a plunger may have a torque resistance of approximately 60 g*cm such that the combined torque resistance in the non-preferred direction of the friction element and plunger may be approximately 150 g*cm. More generally, the torque resistance of the friction element in a non-preferred direction may range between 80 to 95% and/or between 65 to 80% and/or between 30 to 65% and/or between 5 to 30% and/or between 95 to 110% and/or between 110 to 175% and/or between 175 to 300% the torque generated by discharging the drug during delivery. The combined torque resistance in a non-preferred direction of the friction element and the plunger may range for example between 105% to 130% and/or 130% to 170% and/or between 170% to 250% and/or 250% to 500% the torque generated by discharging the drug during delivery. The torque generated discharging the drug during delivery may depend for example on the internal bore of an injection needle and/or on the cross sectional area of the reservoir and/or on the viscosity of the medicine and/or on a pitch of a driving screw. For example, for a 8.65 inner diameter reservoir with a 27G ultra thin wall delivery needle and a drug with viscosity between 30 to 50 cp, and an axial plunger speed of 1-3 cm/min, the axial force discharging the drug may be for example in the range between 0.2-3kgf, the delivery torque discharging the drug during may range for example between 50-150gr*cm torque on the screw. The combined torque resistance in a non-preferred direction of the friction element and the plunger may range for example between 50 to 300*gr.cm. For example as viscosity, the screw pitch, the cross sectional area increase and/or as the needle bore diameter decreases, the delivery torque may increase. Table 1 shows some approximate exemplary values for delivery torque (for example with an 8.65 inner diameter reservoir with a 27G ultra thin wall delivery needle). For example, the delivery torque discharging the drug during may range between 30 to 45 g*cm and/or between 45 to 70 g*cm and/or between 70 to 90 g*cm and/or between 90 to 130 g*cm and/or between 130 to 200 g*cm and/or between 200 to 1000 g*cm.

**Table 1 - examples of delivery torque for discharging the drug (not including friction of the friction element and the plunger with the wall of the reservoir)**

| Torque Generated While Delivery | | | |
|---|---|---|---|
| | Viscosity of Drug | | |
| TSA pich | 1cp | 30cp | 60cp |
| 0.75mm | 49.4g*cm | 74g*cm | 123g*cm |
| 0.85mm | 51g*cm | 86.5g*cm | 127g*cm |
| 1mm | 53g*cm | 86g*cm | 133g*cm |

In some embodiments, tradeoffs are possible. For example, for a higher viscosity drug, a lower pitch driving screw may be used and/or a higher friction anti-rotational pad may be used with a high pitch screw and/or a larger bore needle may be used. For example, the pitch of a driving screw may range between 3mm to 1mm and/or between 1 mm to .8 mm and/or between .8 mm to .6 mm and/or between .6 mm to .3 mm and/or between .3 mm to .2 mm.

In some embodiments, the friction element may be configured to reduce frictional resistance to axial advance of the pushing shaft. For example, the normal force between the friction element and the wall of the reservoir may be increased by tangential movement and/or decreased by axial movement. For example, the ratio of normal force during tangential movement to normal force during axial movement may range between 1.1 to 4 and/or between 4 to 10 and/or between 10 to 100 and/or more than 100. In some embodiments, the ratio of friction coefficient during tangential movement to the friction coefficient during axial movement may range between 1.1 to 4 and/or between 4 to 10 and/or between 10 to 100 and/or greater than 100.

In some embodiments, a friction element may include directional friction surface, for example micro nails and/or an embossed surface pattern and/or slanted hairs and/or scales and/or a non-isotropic molecular structure etc.

In some embodiments, after advancing in and unengaged state, the shaft may be engaged to a plunger.

An aspect of some embodiments of the current invention relates to a connector for an anti-rotational element. Optionally, snap connector connects an anti-rotation friction ring to a plunger driver, for example to a plunger driving shaft thereof. For example, the snap connector may permanently connect a friction ring to the driver. For example, the snap connector may inhibit movement of the anti-rotation element in one or more directions along one or more of six axes (e.g. linear movement in one or more of three axes and/or rotation around one or more of three axes).

In some embodiments, the snap connector may include an interference element. For example, the interference element may interfere with movement of the friction element away from the driver. In some embodiments, the interference element may be flexible and/or the interference element may fit to a complementary flexible element. For example, an interference element may include a flexible portion of a rigid plastic element and/or an elastomeric element. Optionally, a component of the anti-rotational element may be made of elastomer (for example rubber and/or a synthetic elastomer). Optionally a flexible interference element may be built into the elastomer. For example, a friction pad may snap connect to a mount thereof and/or to a plunger driver and/or a shaft therefore. For example, a mount may snap connect to a plunger driver and/or a shaft thereof.

In some embodiments, the friction element may be connected to the plunger driver with a threaded connection and/or a friction connection and/or an interlocking element and/or an interference element. The friction element may be inhibited from moving with respect to the driver in one or more of six linear and/or rotational directions. For example, the friction element may hold the driver to the reservoir during assembly into an injection device and/or during storage and/or shipping. Optionally the friction element may include an anti-rotational element. For example, the anti-rotational element may inhibit rotation of the driver with respect to the reservoir and/or allow linear movement with respect to the reservoir. For example, a threaded driver may drive linear movement.

In some embodiments, an anti rotational element may be inhibited from rotating with respect to a reservoir by friction between the element and an inner wall of the reservoir. In some embodiments, an anti rotational element may be inhibited from rotating with respect to a plunger seal by friction between the element the plunger seal. In some embodiments, the plunger seal may be inhibited from rotating with respect to a reservoir by friction between the plunger seal and an inner wall of the reservoir.

An aspect of some embodiments of the current invention relates to an interface between a plunger driver and a plunger seal of a drug reservoir. For example, the plunger driver may have a surface that abuts against a surface of the plungers seal. For example, a distal surface of the driver may push against a proximal surface of the plunger seal. Optionally, friction, for example between the abutting surfaces, may inhibit rotation of the driver with respect to the plunger seal. For example, an anti-rotation friction element of the driver may abut against the plunger seal and/or a mount thereof. Optionally, the friction element and/or the plunger seal may be formed of elastomer. In some embodiments, a surface and/or a portion thereof may be coated, for example with a friction enhancing compound (e.g. an elastomer and/or a rough surfacing) and/or a friction reducing compound (for example Polytetrafluoroethylene (PTFE)). Alternatively or additionally, the driver may engage to the plunger seal with a threaded connection and/or an interlocking element and/or an interference element.

An aspect of some embodiments of the current invention relates to a friction element retaining a plunger driver to a drug reservoir. Optionally, friction between the friction element and in inner surface of the reservoir inhibits separation of the driver from the reservoir.

An aspect of some embodiments of the current invention related to a vent in a friction element and/or a driver. For example the vent may allow fluid (e.g. gas and/or liquid) to escape the reservoir and/or enter the reservoir as the driver enters and/or exits the reservoir. The vent is optionally formed in the friction element and/or between the friction element and a wall of the reservoir and/or between the friction element and a mount and/or in the mount.

### DETAILED EMBODIMENTS

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Method of facilitating preferred movement of a plunger driver

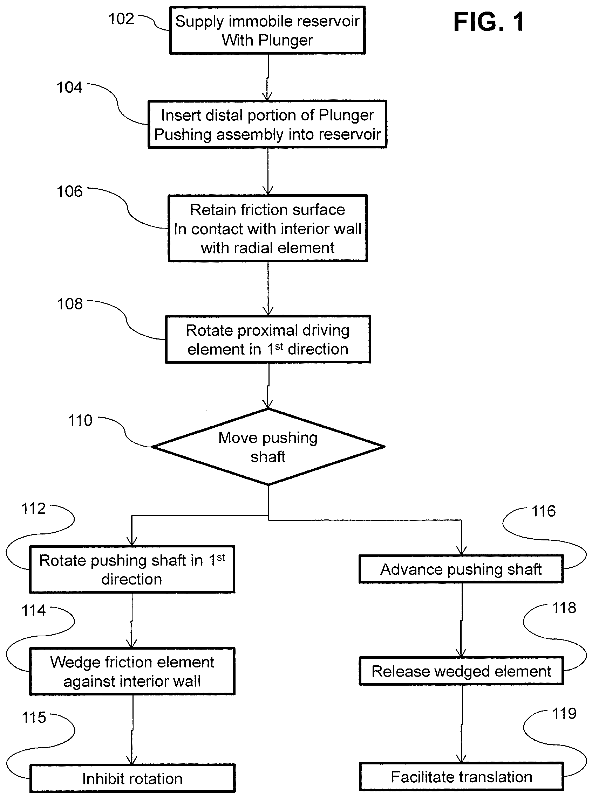

FIG. 1 is a flow chart illustration of a method of facilitating movement of a plunger pushing shaft in accordance with embodiments of the current invention. In some embodiments, a friction element may, for example, prevent the pushing shaft from rotating with respect to a reservoir thereby facilitating relative rotation between the pushing shaft and a driver element. Optionally, whenever the pushing shaft begins to rotate in an inhibited direction, friction between the pushing shaft and the reservoir increases. In some embodiments, friction is reduced when the pushing shaft advances. For example, reduction in friction may reduce a load on a drive system while pushing the plunger and/or discharging a drug.

In some embodiments, an immobile medicine reservoir may be supplied **102.** For example, the reservoir may have a cylindrical cavity with a distal opening and/or a proximal opening. A plunger is optionally inserted in the cylindrical cavity. For example, the plunger may divide between a distal portion of the cavity containing the drug and the proximal portion of the cavity.

In some embodiments, a distal portion of a telescoping plunger pushing assembly may be inserted **104** into the proximal opening of the reservoir. For example, the distal portion of a telescoping plunger pushing assembly may include a pushing shaft and/or a distal portion thereof, a plunger interface and/or a friction element. Optionally a plunger interface of the pushing assembly will engage the plunger when the assembly is inserted **104.** Alternatively or additionally, the plunger interface may remain disengaged from the plunger when the assembly is inserted **104** into the reservoir. In some embodiments, the distal end of the pushing assembly may be inserted **104** until it contacts the plunger. Alternatively or additionally, after insertion **104,** the distal end of the pushing assembly may remain distanced proximally from the plunger.

In some embodiments, a friction surface may be retained **106** in contact with an interior wall of the reservoir and/or with the pushing shaft. For example, when the system is at rest, a radial element may keep at least a minimal normal force between the friction surface and the interior wall of the reservoir. For example, the radial element may be flexible and/or elastically compressible.

In some embodiments, a driving element may be rotated **108** in a first direction. Optionally the driving element may be engaged to the pushing shaft. For example, rotation **108** of the driving element may cause the pushing shaft to move **110.** For example, the pushing shaft may tend to rotate **112** along with the driving element in the first direction. In some embodiments in may be desired to inhibit rotation **112** of the pushing shaft. For example, when telescoping is driven by relative rotation between the driving element and the pushing shaft, rotation **112** of the pushing shaft along with the driving element may be undesirable. Optionally, the friction element may be configured such that rotation **112** of the pushing shaft in the first direction wedges **114** the friction element and/or the radial element between the pushing shaft and the walls of the reservoir. Wedging **114** of the friction element between the driving shaft and the non-rotating reservoir optionally increases a normal force between the friction element and the walls of the reservoir and/or increases friction between the driving shaft and the reservoir and/or inhibits **115** rotation **112** of the pushing shaft.

In some embodiments, a set of screw threads may engage between the driver element and the pushing shaft. For example, rotation **108** of the driver element in the first direction with respect to the pushing shaft may advance **116** the pushing shaft distally into the reservoir. For example, advancing **116** may cause the pushing shaft to approach the plunger and/or cause the interface to engage the plunger and/or cause the plunger to advance distally. Optionally the friction element and/or the radial element is configured such that advancing **116** the pushing shaft releases **118** a wedged element, for example the friction element and/or the radial element. Releasing **118** the wedged element optionally decreases a normal force between the friction element and the walls of the reservoir and/or decreases friction between the driving shaft and the reservoir and/or facilitates **119** advancing of the pushing shaft.

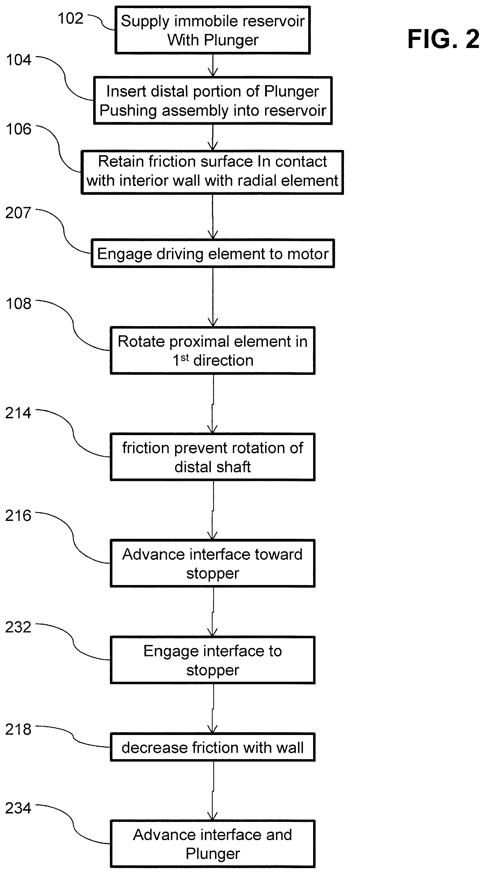

FIG. 2 is a flow chart illustration of a method of driving a plunger in accordance with embodiments of the current invention. Optionally the plunger is driven by an advancing pushing shaft. For example, advancing of the pushing shaft may be motivated by relative rotation of a drive element with respect to a pushing shaft. Tangential friction between a friction element and the reservoir may supply counter rotational torque between the pushing shaft and the reservoir, facilitating relative rotation between the pushing shaft and the driver element. In some embodiments, the pushing shaft may advance in the reservoir while the pushing shaft is unengaged to the plunger. Optionally while the pushing shaft and the plunger are unengaged, the tangential friction between the friction element and the reservoir may be substantially the only force preventing the pushing shaft from rotating along with the rotating element. In some embodiments, the friction element may be configured to produce increased resistance to an undesired movement (for example rotation with the driving element) and/or decreased resistance to a desired movement (for example advancing of the plunger).

In some embodiments, a distal portion of a plunger pushing assembly may be inserted **104** into a proximal opening of a drug reservoir. Optionally the drug reservoir is rotationally immobile. Optionally the drug reservoir includes a plunger separating between a drug in a distal portion of the reservoir and the proximal opening of the reservoir. Optionally, after insertion, a plunger interface of the pushing assembly may remain distanced proximally from the plunger. Optionally, the plunger interface is mounted on a distal portion of the pushing shaft.

In some embodiment, after the distal end of the plunger driving assembly is inserted **104** into the reservoir, a driving element may be engaged **207** to a power source. Alternatively or additionally, the driving element may be engaged **207** to the power source before being insertion **104** of the pushing assembly. For example, the power source may include an electric motor. The power source optionally rotates **108** the driving element in a first direction.

In some embodiments, during rotation **108** of the driving element in the first direction; the pushing shaft may be inhibited **214** from rotating by friction between a friction element and the inner wall of the reservoir. Optionally, rotation **108** of the driving element in the first direction relation to a pushing shaft causes the pushing shaft to telescope and/or move distally with respect to the driving element. For example, the pushing shaft and/or the plunger interface may be advanced **216** distally until it reaches the plunger.

In some embodiments, the plunger interface may engage **232** the plunger. For example, the plunger interface may transfer axial force between the plunger and the pushing shaft. In some embodiments, the plunger interface may limit rotation between the plunger and the pushing shaft. For example, after engagement of the plunger, friction between the plunger and the interior wall of the reservoir may prevent rotation of the pushing shaft in addition to and/or in place of the friction element. Alternatively or additionally, the plunger interface may allow rotation of the pushing shaft with respect to the plunger. Optionally, allowing rotation of the pushing shaft with respect to the plunger may reduce rotational stresses on the plunger.

In some embodiments, when the pushing shaft is advancing and/or when the plunger interface is engaged to the plunger, friction between the friction element and the wall of the reservoir may be reduced **218.** Reducing the friction may make it easier to advance **216** the plunger driver and/or advance **234** the plunger.

### Block diagram of an anti-rotation system

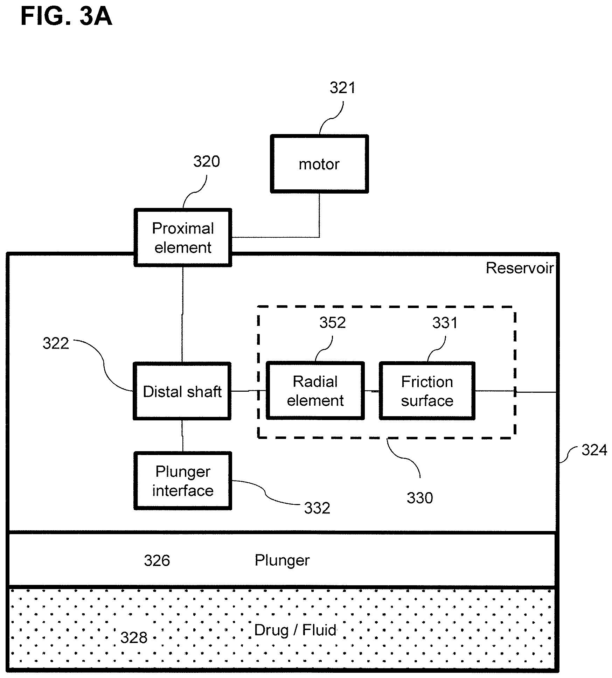

FIG. 3A is a block diagram illustration of a drug reservoir and an assembly for driving a plunger in accordance with embodiments of the current invention. For example, plunger driving assembly may include a variable friction assembly **330** and/or a telescoping assembly including a proximal element **320** and/or a distal shaft **322** and/or a plunger interface **332.** The telescoping assembly optionally telescopes via rotation of proximal element **320** (for example a driver element) with respect to distal shaft **322** (for example a plunger pushing shaft). For example, proximal element **320** may be rotated with respect to a drug reservoir **324** and/or distal element **322** is hindered from rotating with respect to drug reservoir **324.** Optionally, distal element **322** is hindered from rotation by friction with the walls of reservoir **324.** For example, friction with the walls of drug reservoir **324** may be supplied by variable friction assembly **330.** For example, variable friction assembly **330** may have high resistance to rotation and/or tangential movement with respect to the drug reservoir and/or low resistance to axial translation with respect to the drug reservoir. Optionally, a motor **321** rotates proximal element **320** with respect to reservoir.

In some embodiments, variable friction assembly **330** may include a radial element **352** and/or a friction surface **331.** Optionally radial element **352** links friction surface **331** to distal shaft **322.** Optionally radial element **352** retains friction surface **331** in contact with an interior wall of reservoir **324.**

In some embodiments, radial element **352** maintains a variable normal force between friction surface **331** and the interior wall of reservoir **324.** For example, radial element **352** may be wedged between the interior wall of reservoir **324** and shaft **322** such that tangential movement of friction surface **331** in at least one direction causes compression of radial element (optionally increasing the normal force). Alternatively or additionally, radial element **352** may be wedged between the interior wall of reservoir **324** and shaft **322** such that axial movement of friction surface **331** in the distal direction causes release of compression of radial element (optionally decreasing the normal force). For example, the radial element may include one or more of a sprag, a virtual sprag, a beam having a preferred bending direction, a clutch dog, a wedge, a prop and/or a virtual beam having a preferred bending direction.

In some embodiments radial element **352** may be wedged by movement in any of a plurality of directions. For example, radial element **352** may include a trapezoidal sprag that is wedged on one side by movement in one direction and on the other side by movement in an opposite direction. Alternatively or additionally, friction assembly **330** may include multiple anisotropic sprags and/or beams oriented in different directions to cause increased friction in more than one direction. For example, friction assembly **330** may have increased friction resistance to rotation around an axis in both rotational directions and/or have decreased friction to linear movement in one or more directions.

In some embodiment, friction surface **331** may have an anisotropic friction coefficient. For example, the friction coefficient may be high in one of the tangential directions optionally causing high friction resistance to rotation of the distal pushing shaft with respect to the reservoir in at least one direction and/or low friction resistance to movement distally with respect to the drug reservoir. Alternatively or additionally, the friction surface may have an isotropic coefficient of friction.

In some embodiments, proximal element **320** may be proximal to, partially inserted into and/or fully inserted into a proximal opening reservoir **324.** In some embodiments, distal shaft **322** may be proximal to, partially inserted into and/or fully inserted into a proximal opening reservoir **324.**

In some embodiments reservoir **324** may contain a fluid, for example a drug **328.** For example, drug **328** may be located in distal portion of reservoir **324.** Optionally a plunger seal **326** may seal off and/or separate between drug **328** and the proximal opening of the reservoir and/or the plunger pushing assembly.

In some embodiments when plunger interface **332** is engaged to plunger seal **326** it may supply a one-way, two-way or multi-dimensional connection between pushing shaft **322** and plunger seal **326.** For example, plunger interface **332** may include a ram that supplies compression force between shaft **322** and plunger seal **326** for pushing plunger distally, but supplies small and/or negligible tensile force for pulling plunger seal **326** proximally. This ram may have friction surface with the plunger and/or partially intrude into the plunger. Alternatively, plunger interface **332** may include a snap, screw and/or interference element that supplies compression force between shaft **322** and plunger seal **326** for pushing plunger distally and also supplies tensile force for pulling plunger seal **326** proximally. In some embodiments, interface **332** transmits torque between plunger seal **326** and shaft **322.** Alternatively or additionally, interface **332** may not transmit significant torque; for example interface **332** may include a pivot that allows shaft **322** to rotate independently of plunger seal **326.**

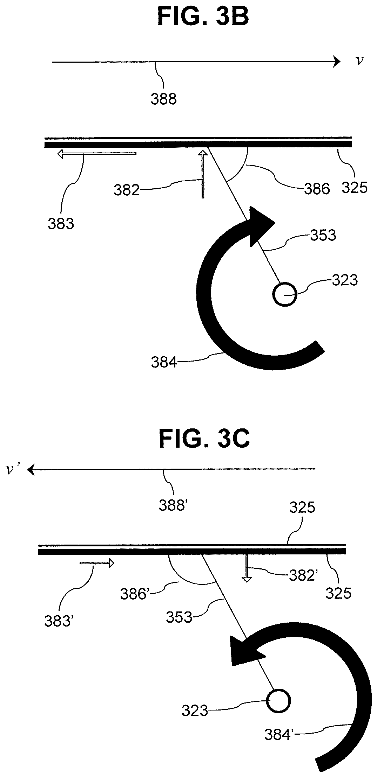

FIG. 3B is a schematic diagram of a sprag **353** in accordance with an embodiment of the current invention. For example, in FIGs. 3B and 3C, sprag **353** is in contact with a surface **325.** For example, in FIG. 3B surface **325** is moving in an inhibited direction indicated by arrow **388.** Optionally, when surface **325** is moving in an inhibited direction, the angle of attack **386** between sprag **353** and the direction of movement will be less than 90 degrees. For example, the angle of attack may range between 89 to 85 degrees and/or between 85 to 70 degrees and/or between 70 to 45 degrees and/or between 45 to 0 degrees The sprags are anisotropic. An angle of attack differs on a leading and/or trailing edge. The movement of surface **325** optionally causes a rotational moment **384** on sprag **353** and/or wedges sprag **353** between the outer wall of a shaft **323** and surface **325.** When movement is in the inhibited direction, rotational moment **384** and/or wedging optionally produces an increase **382** in the normal force of sprag **353** against surface **325.** In some embodiments, when movement is in the inhibited direction, rotational moment **384** and/or wedging may produce an increase a force **383** of friction of sprag **353** against surface **325.**

FIG. 3C is a schematic diagram of a sprag **353** in accordance with an embodiment of the current invention. For example, in FIG. 3C surface **325** is moving opposite an inhibited direction as indicated by arrow **388'.** Optionally, when surface **325** is moving opposite an inhibited direction, the angle of attack **386'** between sprag **353** and the direction of movement will be greater than 90 degrees. For example, the angle of attack may range between 91 to 95 degrees and/or between 95 to 120 degrees and/or between 120 to 135 degrees and/or between 135 to 180 degrees. The movement of surface **325** optionally causes a rotational moment **384'** on sprag **353** and/or releases wedging of sprag **353** between the outer wall of a shaft **323** and surface **325.** When movement is opposite the inhibited direction, rotational moment **384'** and/or releasing the wedging optionally produces a decrease **382'** in the normal force of sprag **353** against surface **325.** In some embodiments, when movement is opposite the inhibited direction, rotational moment **384'** and/or releasing the wedging may produce a decrease in a force **383'** of friction of sprag **353** against surface **325.**

### Independent anti-rotation sprags

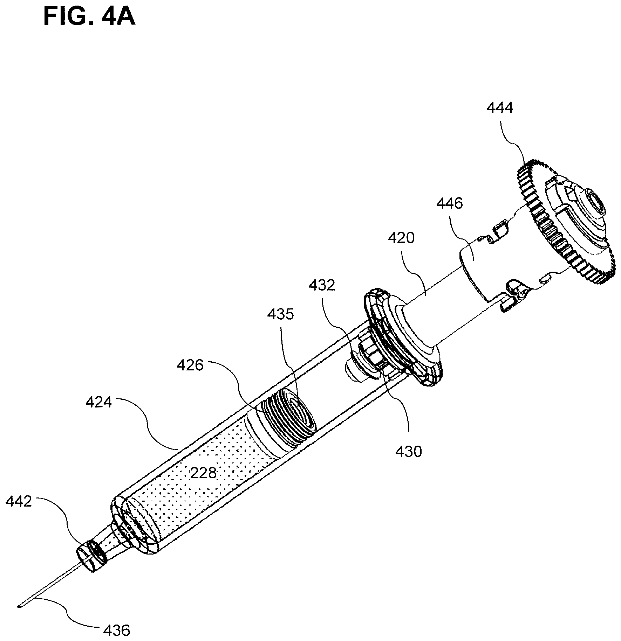

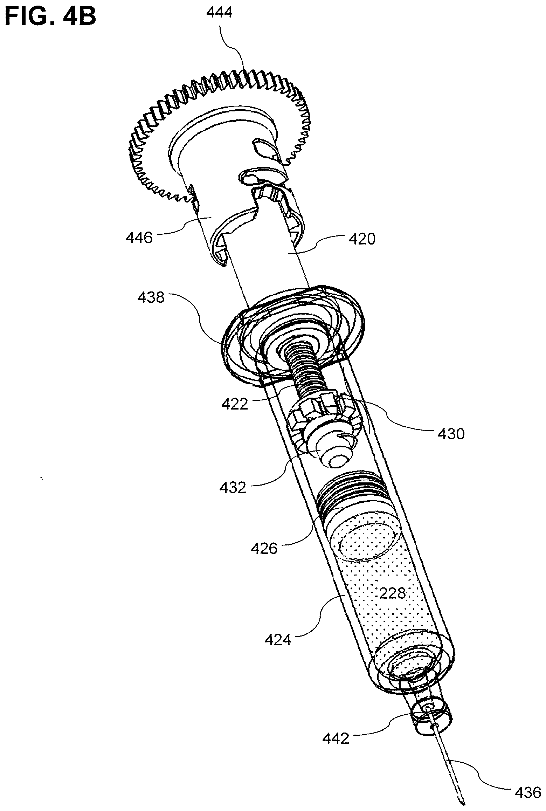

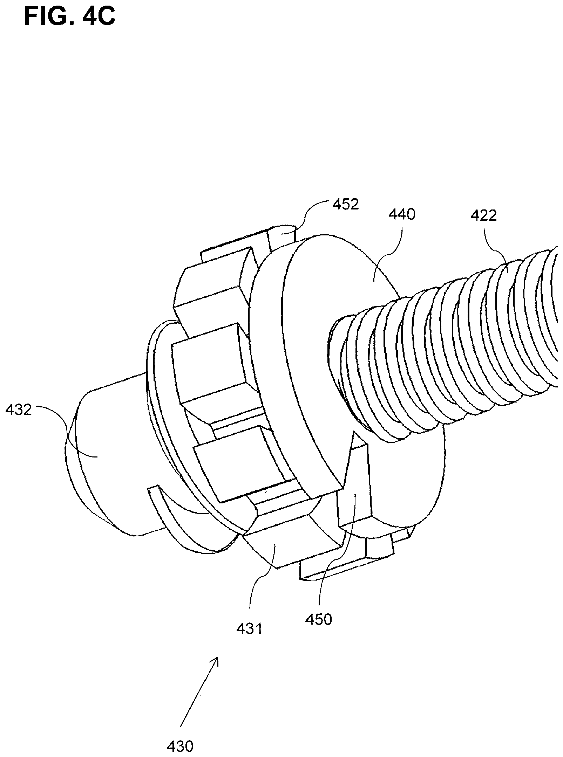

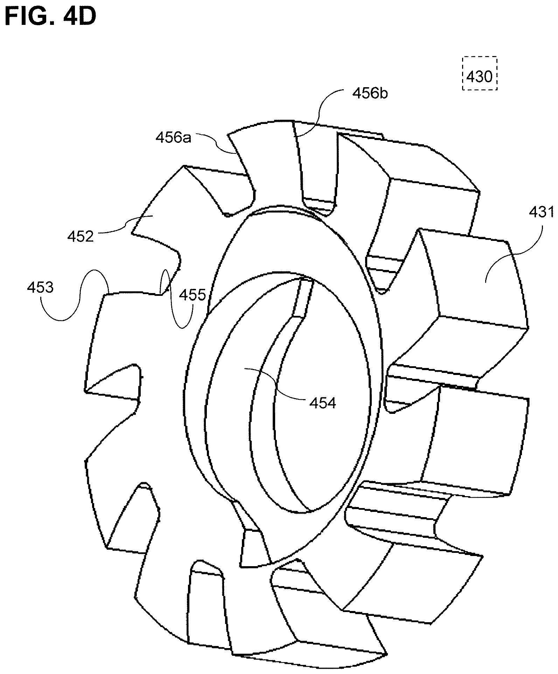

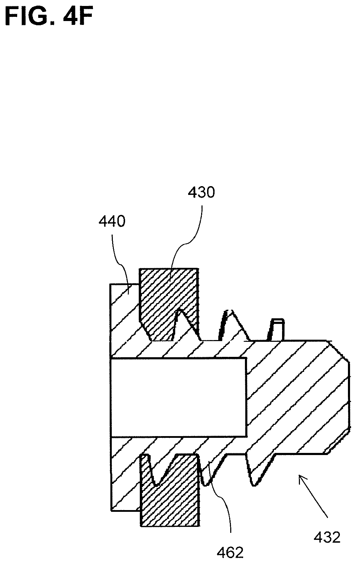

FIGs. 4A, 4B, 4C, 4D and 4F are drawings of a plunger driving assembly and drug reservoir with a screw-in plunger interface in accordance with embodiments of the current invention. In some embodiments, a multi sprag arm variable anti-rotation friction element **430** may supply rotational resistance to a pushing shaft (for example see pushing shaft **422** FIG. 4B). The anti-rotational friction may facilitate relative rotation and/or telescoping of pushing shaft **422** with respect to a driver (for example driver **420**). For example pushing shaft **422** may be threaded and driver **420** may include a threaded element, for example a threaded shaft and/or a nut.

FIG. 4A illustrates a plunger driving assembly initially inserted into a proximal opening of a drug reservoir **424** in accordance with an embodiment of the current invention. In some embodiments, distal portion of the plunger driving assembly including for example a plunger interface **432.** For example, interface **432** fits into and/or threadably connects to a cavity **435** in plunger seal **426.** Additionally or alternatively, a driving assembly may include a variable resistance friction element **430** and/or a distal portion of pushing shaft **422.** Optionally when the plunger driving assembly is inserted, a plunger interface **432** remains distal to and/or disengaged from a plunger seal **426.**

In some embodiments, a transmission gear **444,** driver **420,** a needle retraction lock **446** and a part of the proximal shaft **422** are located outside and/or proximal to reservoir **424.** Optionally the distal end of reservoir **424** includes a distal opening and a needle mount **442.** The distal opening is optionally connected to a hypodermic needle **436.** Optionally the distal portion of reservoir **424** is filled with a drug **228.** Plunger seal **426** separates and/or seals reservoir **424** between medicine **228** and a proximal portion and/or the proximal opening of the reservoir.

In some embodiments, a drug cartridge may include an indicator. Optionally the indicator may include a part of a plunger seal **426** and/or the plunger driver assembly. Optionally the indicator is visible to the user through a portion of the wall of reservoir **424,** which may be for example transparent, tinted and/or translucent. For example, friction element **430** may indicate to a user the location and/or movement of the plunger driving assembly. Plunger interface, **432** may indicate when the driver assembly starts to push plunger seal **426** (e.g. the beginning of discharge). For example, interface element **432** may have a different color than plunger seal **426** and/or friction pad **430.** For example, interface element **432** may be red while plunger seal **426** and/or friction pad **430** are blue. When element **432** has not yet entered cavity **435,** the users sees red, which indicates that discharge has not yet started. Alternatively or additionally, an indicator may include a part that fits into cavity **432** when the driver contacts seal but does not engage to plunger seal **426.**

FIG. 4B illustrates a plunger driving assembly with the telescoping assembly beginning to open, advancing plunger interface **432** towards plunger seal **426** in accordance with an embodiment of the current invention. In some embodiments, after insertion, the telescoping assembly may be extended to advance interface **432** toward plunger seal **426** and/or to engage interface **432** to plunger seal **426.**

In some embodiments, interface **432** is advanced by rotating transmission **444** and/or retraction lock **446** and/or driver **420** with respect to reservoir **424.** Driver **420** is optionally threadably engaged to pushing shaft **422.** In some embodiments, friction between friction element **430** and the inner walls of reservoir **424** inhibits rotation of pushing shaft **422** such that rotating driver **420** results in relative rotation between driver **420** and pushing shaft **422.** Relative rotation of driver **420** and pushing shaft **422** optionally advances pushing shaft **422** and/or interface **432** towards plunger seal **426.**

In some embodiments, interface **432** advances until it contacts plunger seal **426.** When interface **432** contacts plunger seal **426,** interface **432** may become linearly linked to plunger seal **426** (for example in that further linear advancement of interface **432** advances plunger seal **426**) and rotationally independent of plunger seal **426** (in that rotation of interface **432** does not rotate plunger seal **426**). In some embodiments, distal advancement optionally stops due to the normal force between interface **432** and plunger seal **426** and/or friction force between plunger seal **426** and the walls of reservoir **424** and/or the normal forces between drug **228** and plunger seal **426.** Optionally rotation of driver **420** while interface **432** does not advance overcomes friction between friction element **430** and the walls of reservoir **424** and/or causes rotation of interface **432.** For example, rotation of interface **432** may screw it into plunger seal **426.**

In some embodiments, when interface **432** is fully screwed into plunger seal **426,** interface **432** becomes rotationally linked to plunger seal **426** (for example in that further rotation of interface **432** rotates plunger seal **426**). In some embodiments, rotation of interface **432** optionally stops due to the combined frictional resistance to rotation of interface **432** and plunger seal **426** with respect to the walls of reservoir **424.** Optionally rotation of driver **420** while interface **432** does not rotate overcomes resistance to axial motion and/or plunger seal **426** begins to move axially and/or discharges the drug from the distal opening of the reservoir (for example through needle **436**). In some embodiments, reservoir **424** may include a flange **438.** For example, flange **438** may extend out from a rear portion of reservoir **424.**

FIG. 4C is a close up perspective view of a distal end of a pushing shaft including a plunger interface **432** and a friction element **430** in accordance with an embodiment of the current invention. In some embodiments, an anti-rotation assembly may include a vent **450.** For example, vent **450** may allow air to escape the zone between plunger seal **426** and interface **432** for example as interface **432** approaches plunger seal **426.**

In some embodiments, a stabilizing base **440** may limit proximal movement of friction element **430** and/or determine the axial relationship of interface **432** and/or shaft **422** to friction element **430** and/or direct bowing and/or buckling of a radial element **452.** For example, base **440** prevents radial elements **452** from buckling proximally and/or disengaging from the walls of reservoir **424.** Optionally, there is a friction surface **431** on an external end of at least on radial element **452.** For example, friction surface **431** may be held in contact with an interior wall of the reservoir.

FIG. 4D is a close up perspective view of a friction element **430** in accordance with an embodiment of the current invention. Element 430 is a flexible disk shaped element with independent flexible radial elements **452,** for example sprags. Each sprag includes a friction surface **431** for contacting the walls of reservoir **424.** Each sprag is tilted off the radial direction towards the direction of inhibited movement. Optionally friction surface **431** is on the outer end of the each radial element **452.** For example, the outer end of each radial element **452** spread as it radiates outward and/or the outer end (head **453**) of each sprag may be wider than its central end (stem **455**).

In some embodiments, radial elements **452** are angled against an inhibited direction of rotation. For example, the inhibited direction of rotation may be the direction of rotation of driver **420** for extension of the telescoping assembly. Optionally, when driver **420** is rotating in a direction to expand the telescoping assembly (and/or advance pushing shaft **422)** and frictional element **430** begins to rotate in the direction of rotation of driver **420,** sprag type radial elements **452** get wedged against the walls of reservoir **424.** Optionally, when driver **420** is rotating in a direction to expand the telescoping assembly (and/or advance pushing shaft **422)** and frictional element **430** begins to rotate in the direction of rotation of driver **420** friction is increased between frictional element and reservoir **424** inhibiting the rotation of shaft **422** and/or facilitating relative rotation between driver **420** and shaft **422** and/or facilitating advancement of shaft **422.** The sprags 452 are anisotropic. For example, the angle of attack of a leading edge **456a** may be steeper than the angle of attack of a trailing edge **456b.** For example, the mean angle of attack of sprag **452** may range between 80 and 90 degrees while the mean difference between the leading **456a** and trailing **456b** edges may range between 1 and 9 degrees. For example, the mean angle of attack of a sprag may range between 60 and 80 degrees while the mean difference between the leading and trailing edges may range between 1 and 40 degrees. For example, the mean angle of attack of a sprag may range between 20 and 60 degrees while the mean difference between the leading and trailing edges may range between 1 and 70 degrees.

In some embodiments, friction element **430** may be made of rubber, elastomer and/or silicone for example of hardness shore 00 scale approximately 50. For example, the hardness may range between shore 00 scale 10 to 30 and/or 40 to 60 and/or 60 to 100 and/or shore A scale 60 to 100. Optionally, the unstressed diameter of friction element **430** is slightly larger than the inner diameter of reservoir **424.** For example, the unstressed outer diameter of element **430** may be approximately **9.**1mm while the inner diameter of reservoir **424** may be approximately 8.65 mm. Optionally the inner diameter of a reservoir may range between 1 to 4 mm and/or between 4 to 7 mm and/or between 7 to 10 mm and/or between 10 to 15 mm and/or between 15 to 30mm. Optionally the outer diameter of a friction element may range between 100% to 103% and/or 103% to 107% and/or 107% to 120% and/or 120% to 150% the internal diameter of the reservoir. In some embodiments, friction element **430** may have a closed diameter at the base (stem **455**) of sprags **452** of approximately 6.3 mm and/or the length of sprags **452** (in the radial direction) may be approximately 1.4 mm. For example, the thickness (along the length of reservoir **424**) of element **430** and/or sprags **452** may be 2 mm and/or radial length of the head of sprags **452** may be approximately 1.65 mm and/or the surface **431** area of each sprag **452** in contact with the inner wall of reservoir **452** may be approximately 2+1.65=3.3 mm². In some embodiments, the length of a sprag element may range for example between 1 and 10% the diameter of the reservoir and/or between 10 to 20% the diameter of the reservoir and/or between 20 to 40% the diameter of the reservoir and/or between 40 to 70% the diameter of the reservoir and/or between 70 to 100% the diameter of the reservoir. In some embodiments, the width of a friction element may range for example between 1 to 5% and/or between 5 to 15% and/or between 15 to 25% and/or between 25 to 50% and/or between 50 to 75% and/or between 75 to 150% and/or between 150 to 600% the diameter of the friction element.

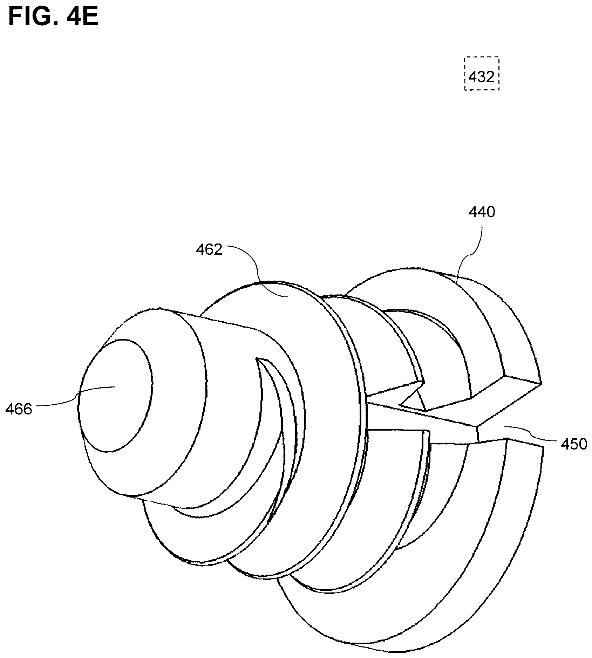

In some embodiments, friction element **430** may include a mount for connection to pushing shaft **422** and/or to interface **432.** For example, shaft mount **454** of friction element **430** includes an internally threaded aperture that threadably mates to an external threads **462** of plunger interface **432** (see for example FIG. 4E).

FIG. 4E is a close up perspective view of plunger interface **432** in accordance with an embodiment of the current invention. For example, interface **432** is optionally a hard element (for example made of molded plastic) with a tapered screw thread **462** for attaching to plunger seal **426** and/or friction element **430.** Optionally, interface **432** includes a tapered distal face **466** and/or a proximal base **440** and/or a vent **450.** Optionally vent **450** includes a slit in base **440.** Optionally, slit **450** is positioned to align with a space between radial elements **452.** Alternatively or additionally, a friction element may include a vent. For example, when the friction element is mounted on interface **432,** the vent of the friction element may be aligned with vent **450.**

In some embodiments, friction element **430** may include a mount for connection to pushing shaft **422** and/or to interface **432.** For example, shaft mount **454** of friction element **430** includes an internally threaded aperture that threadably mates to an external threads **462** of plunger interface **432** (see for example FIG. 4E).

FIG. 4F is a cross sectional view of friction element **430** mounted on an adapter **432** in accordance with an embodiment of the present invention. For example, screw threads **462** are smaller at the insertion point (the distal end) and grow as one moves proximally. The rear (proximal) face of the teeth is optionally at a sharper angle (for example between 70 to 95 degrees from the axis of adaptor **432)** than the front (distal) face of the teeth (which range for example at an angle between 115 to 145 degrees from the axis of adaptor **432).**

### Linear motion sensitive sprags

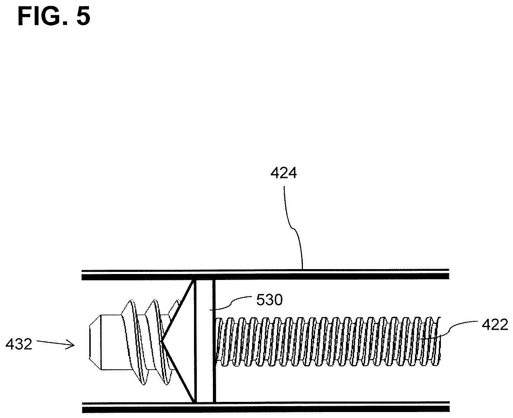

FIG. 5 is a perspective drawing of a plunger pushing rod and friction element for facilitating forward linear motion in accordance with embodiments of the current invention. Optionally, friction element **530** is angled backwards (for example proximally). For example, advancement of shaft **422** the angle of attack of element **530** would be greater than 90 degrees. Optionally advancement of shaft would reduce the normal force and/or the friction between friction element **530** and the wall a reservoir **424.** Optionally, when shaft **422** is rotating but not advancing, the friction between element **530** and reservoir **424** is high, inhibiting rotation. Optionally as shaft **422** advances in reservoir **424,** the shape of element **530** causes a reduction in friction facilitating the advancement.

### Friction disk with anti-rotation beam

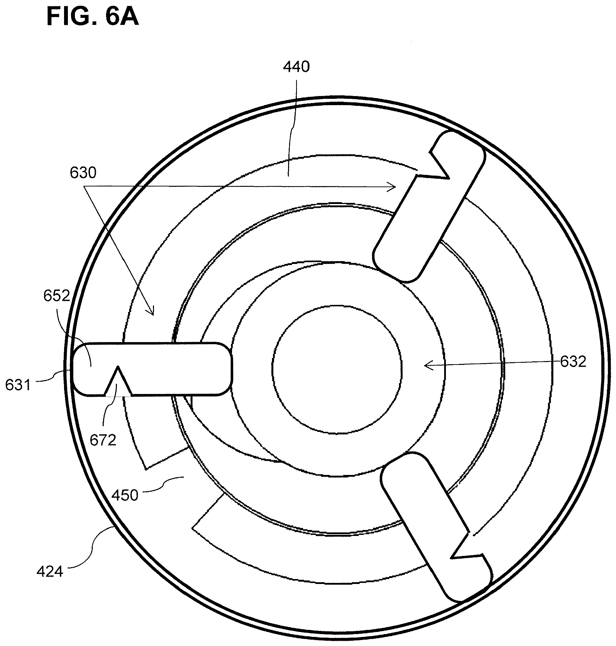

FIG. 6A is cross sectional view of a plunger pushing rod including flexible beam anisotropic friction elements in accordance with embodiments of the current invention. In some embodiments, a friction element **630** may include an anisotropic bending beam **652.** For example, when beam **652** is bent in an inhibited direction it may not bend away and/or it may get wedged between a plunger interface **632** and the walls of reservoir **424.** For example, when beam **652** is bent in a non-inhibited direction, beam **652** may bend out of the way and/or reduce friction. In FIG. 6A elements **652** are illustrated in a relatively unstressed state.

In some embodiments, a beam may have anisotropic compressibility and/or extensibility. For example, beam **652** has a cut out section **672.** The side of the beam with the cut out extends and/or compresses more easily than the opposite side.

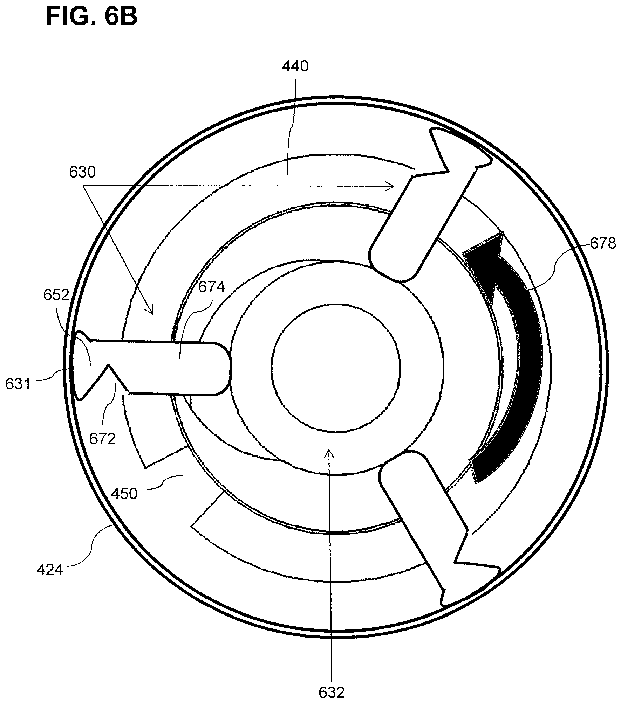

FIG. 6B illustrates element **652** in a stressed state due to rotation of interface **632** in an inhibited direction **678** in accordance with an embodiment of the present invention. Optionally, when element **652** is stressed, cut out section **672** may tend to expand and/or contract while uncut side of the beam may tend to buckle and/or bend. Optionally stress due to rotation in the inhibited direction **678** causes cut out section **672** to open wedging surface **631** against reservoir **424** increasing a normal force and/or a friction between surface **631** and reservoir **424.**

### Pressure sensitive friction element

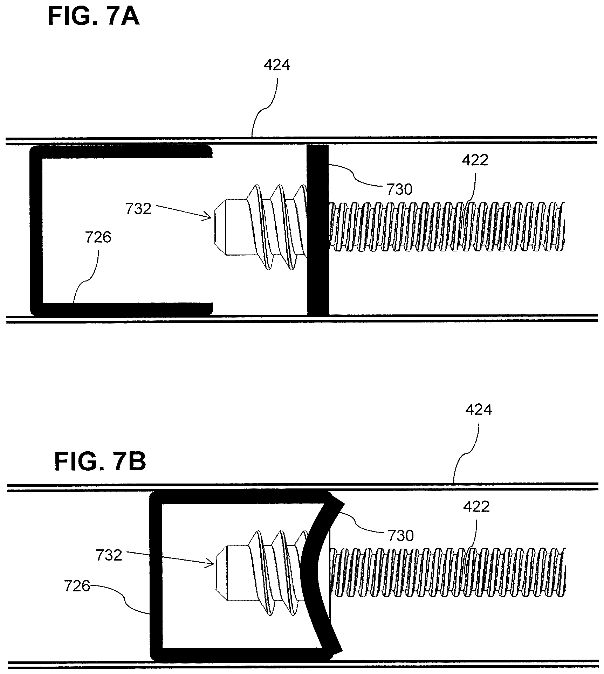

FIGs. 7A-7B are side views of a plunger pushing assembly with reduced friction when engaged to a plunger in accordance with embodiments of the current invention. For example, a friction element **730** may be sized to contact a wall of a drug reservoir and/or produce friction with the wall inhibiting rotation of a plunger pushing shaft **422** and/or a plunger interface **732.** Optionally, when interface **732** engages a plunger **726,** friction between element **730** and the reservoir may be reduced and/or the resistance to advancement of the plunger may be reduced.

FIG. 7A illustrates plunger interface **732** and friction element **730** while disengaged from a plunger in accordance with an embodiment of the current invention. In some embodiments, shaft **422** may be advanced through reservoir **424** by rotation of a driver relative to shaft **422.** As interface **732** approaches plunger **726,** friction element contacts the interior walls of reservoir **424** and/or inhibits rotation of shaft **422** and/or facilitates the relative rotation of the driver with respect to shaft **422.**

FIG. 7B illustrates plunger interface **732** and friction element **730** while engaged to a plunger in accordance with an embodiment of the current invention. In some embodiments, when interface **732** engages plunger **726,** an element (for example the hard side walls of the plunger and or a bowl shaped and/or annular projection) deform friction element **730.** Deformation of friction element **730** optionally reduces and/or eliminates contact between friction element and reservoir **424.** For example, deformation of friction element **730** may reduce and/or substantially nullify friction between element **730** and reservoir **424.** Optionally, when plunger interface **732** is engaged with plunger **726,** friction between plunger **726** and reservoir **424** may inhibit rotation of shaft **422** and/or facilitate advance of shaft **422** and/or plunger **726.** In some embodiments, reduction of the friction between element **730** and reservoir **424** may reduce the load on a plunger pushing assembly. For example, interface **732** may be engaged to plunger **726** while a device is discharging a drug. Reduction of friction between element **732** and reservoir **424** optionally reduces the load on the plunger pushing system while discharging the drug.

In some embodiments advancement of the plunger pushing assembly may proceed through a few friction stages and/or configurations. For example, while interface **732** is distanced proximally from plunger **726,** the resistance to rotation caused friction between element **730** and reservoir **424** may be enough to prevent rotation of shaft **422.** With shaft **422** rotationally immobile, rotation of a driver may produce relative rotation between the driver and shaft **422** and/or advancement of shaft **422.**

In some embodiments, shaft **422** advances until interface **732** contacts the proximal side of plunger **726.** Once interface **732** contacts the proximal side of plunger **726** rotation of the driver and resistance to rotation by friction element **730** may be enough to overcome resistance to advance of plunger **726.** Subsequently, interface **732** is driven forward, driving forward plunger **726** and discharging the medicine. Alternatively or additionally, when interface **732** contacts the proximal side of plunger **726,** resistance to advance of plunger **726** may be enough to stop advance of shaft **422.** While resistance of plunger **726** prevents advance of interface **732,** torque optionally overcomes rotation resistance of element **730.** For example, overcoming rotational resistance may cause interface **732** to rotate and/or be screwed into the proximal end of plunger **726** and/or engage plunger **726.**

In some embodiments, once interface **732** is engagement of interface **732** to plunger **726** may prevent rotation of interface **732** with respect to plunger **726.** Friction between plunger **726** and reservoir **424** may prevent further rotation of shaft **422,** facilitating relative rotation of shaft **422.** Prevent rotation of interface **732** optionally facilitates rotation a driver in relation to shaft **422** optionally facilitating further advance of plunger seal **426.**

### Plunger interface

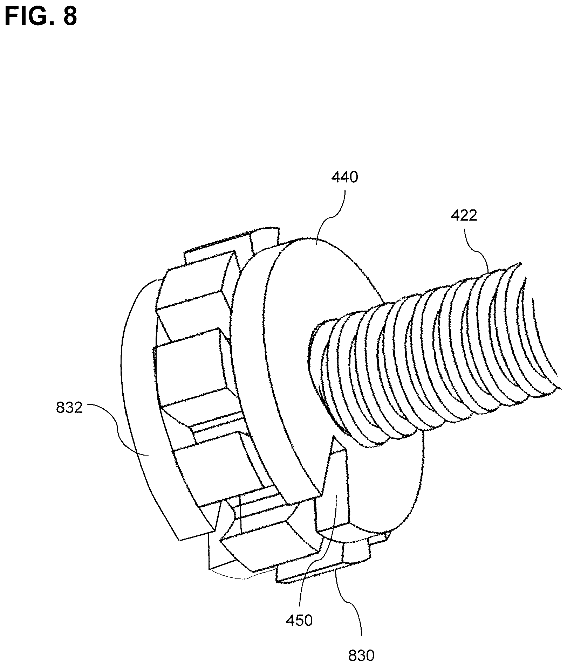

FIG. 8 is a perspective view of a plunger pushing rod including a sprag friction elements and a ram plunger interface in accordance with embodiments of the current invention. In some embodiments, a plunger interface will transfer linear forces between a plunger and a pushing shaft **422.** Optionally the interface will transfer limited or negligible rotational torque between the plunger and the pushing rod. For example a ram **832** may push a plunger along an axis of a drug reservoir (for example distally). Ram **832** may transfer little or no torque around the axis. For example, embodiments, ram **832** may not be rotationally fixed to shaft **422** and/or to the plunger. Torque is optionally transferred between shaft **422** and the plunger by friction. Alternatively or additionally, there may be a rotating element (for example a pivot and/or an axel) between shaft **422** and ram **832** limiting transfer of torque. Optionally, rotational resistance of friction element **830** will be high enough to prevent a driver from rotating shaft **422** under the dual load of linear resistance of friction element **830** and the plunger (resistance to linear movement of the plunger may include for example friction between the plunger and the reservoir and/or resistance to discharge of the drug).

### Friction disk with anti-rotation sprag ribs

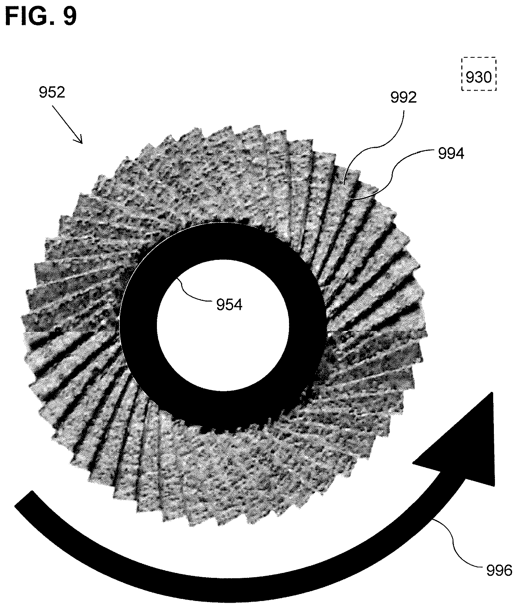

FIG. 9 is a perspective view of a friction element **930** including virtual sprag friction elements in accordance with embodiments of the current invention. In some embodiments, a radial element **952** may be constructed of angled layers of connected sheets **992.** Sheets **992** are optionally angled toward the inhibited direction **996** of rotation. When the periphery of element **930** are in frictional contact with an immobile reservoir wall, rotation of element **930** in the inhibited direction **996** optionally spreads sheets **992** and/or increase a surface area of friction. Alternatively or additionally Rotation in the inhibited direction **996** pushes against joints **994** which are virtual sprag elements that are optionally stiffer than the sheets, for example increasing a normal force between friction element **930** and a wall of a reservoir. Alternatively or additionally virtual sprag elements may include a thickened rib angled against the inhibited direction and/or a rib of a stiffer material than the rest of the disk angled against the direction of inhibited movement. Friction element **930** optionally includes a shaft mount **954.**

### Friction element with rollers

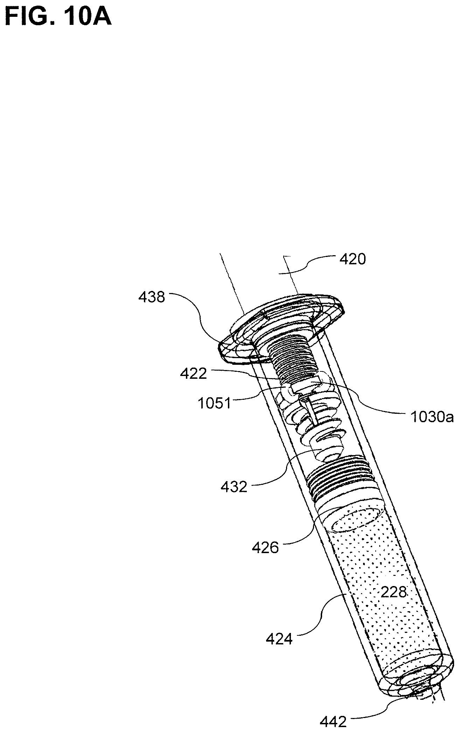

FIG. 10A is a perspective view of a friction element **1030a** including rolling element **1051** in accordance with embodiments of the current invention. For motion in the preferred direction, rolling element **1051** may roll, allowing motion with minimal friction whereas for motion in the non-preferred direction. For example, friction element **1030a** may move axially inside reservoir **424** with a friction force of a hundredth or less than the force necessary to rotate element **1030a** around the axis of reservoir **424.** For example, a rolling element may be round and/or spherical and/or cylindrical and/or tapered. A rolling element may include a wheel and/or an axel and/or a bearing and/or a race and/or a cage.

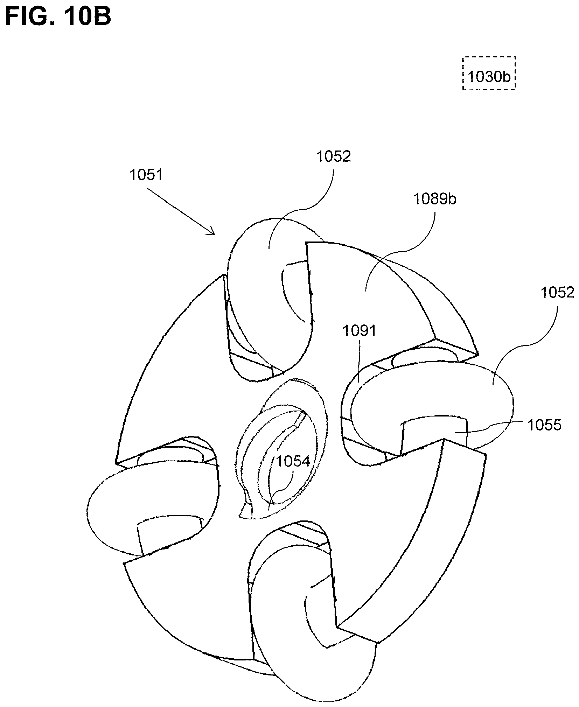

In some embodiments, for example as illustrated in FIG. 10B, rolling element **1051** may include a wheel **1052.** For example, during movement in a preferred direction, for example as element **1030b** moves linearly along the axis of reservoir **424,** wheel **1052** may rotate around an axel **1055.** Optionally, a rolling element may be inside an indentation and/or a ball cage **1091.** For example, cage **1091** optionally includes an indentation in a connecting element **1089b.** For example, connecting element **1089b** may include a disk and/or cylindrical connector between a drive screw and/or a plunger adapter. Alternatively or additionally, friction element may be built into a driving element and/or a plunger adapter. For example, a wheel and/or an axel and/or a race and/or a cage may be built into the plunger adapter and/or the drive element.

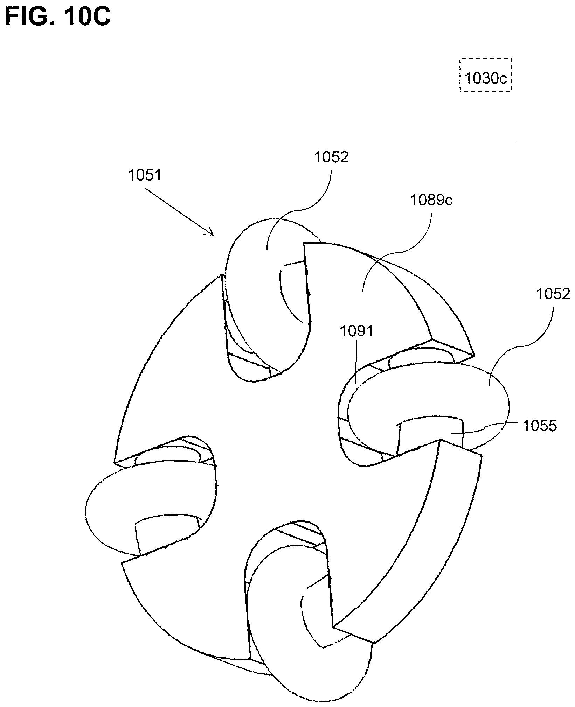

Some embodiments of an anti-rotational element **1030b,** a connecting element **1089b** may include a shaft mount **1054,** for example as illustrated in FIG. 10B. Additionally or alternatively, in some embodiments of an anti-friction element **1030c,** a connecting element 1089c may not have a shaft mount, for example as illustrated in FIG. 10C. For example, connecting element fit between a driving shaft and/or a plunger.

### Friction element preventing rotation of reservoir

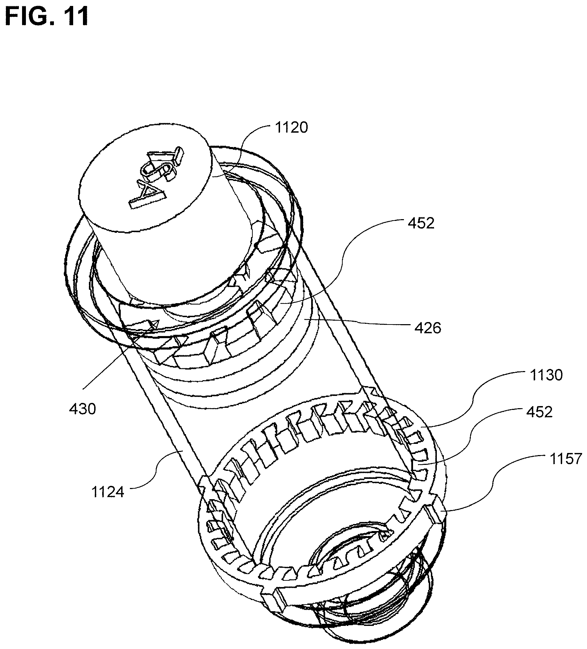

FIG. 11 is a perspective view illustration of a drug reservoir with an external anti-rotational friction element in accordance with an embodiment of the present invention. Optionally, an external friction element **1130** resists rotation of the reservoir with respect to a housing of a drug delivery device. For example, friction element **1130** has anisotropic friction with higher frictional resistance against movement in a non-preferred direction than in a preferred direction. For example, sprags **452** have enhanced friction to resist rotation of reservoir **1124** in a non-preferred direction. Optionally, reservoir can easily be slid longitudinally into element **1130,** but once in place is difficult to move in a non-preferred direction (for example to rotate against sprags **452** and/or to remove reservoir **1124).**

In some embodiments, element **1130** may have the form of a ring surrounding reservoir **1124.** Optionally sprags **452** radiate inward from the ring towards the outer wall of reservoir **1124.**

In some embodiments, a system may include two directionally preferential friction elements. For example, friction element **430** may prevent rotation of a plunger driving shaft. For example, element **430** facilitates movement of a plunger driver by a shaft driver **1120.** In some embodiments, sprags **452** of internal friction element **430** are oriented in the opposite direction from sprags **452** of external friction element **1130.** For example, friction element **430** may prevent a plunger driver from rotating by applying a first torque in a first non-preferred direction (non-preferred with reference to inner element **430)** to an inner wall of reservoir **1124.** Optionally, element **1130** may prevent rotation of reservoir **1124** by providing an opposite torque to the outer wall of reservoir **1124.** The second torque may inhibit rotation of reservoir **1124** in a second non-preferred direction. In some embodiments, the first and second non-preferred directions may be opposite in orientation.

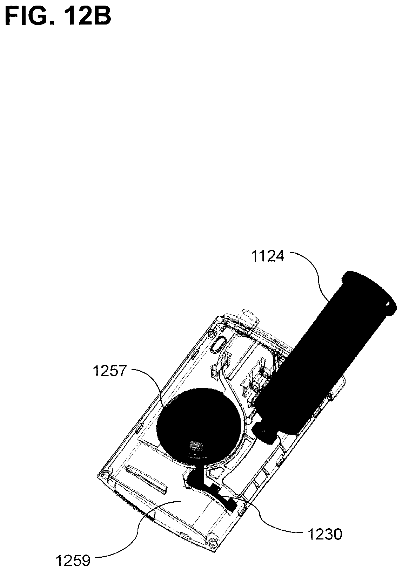

In some embodiments, external friction element **1130** may include anchors **1157** to hold it in a drug delivery device. For example, anchors **1157** are protrusions molded into element **1130** that fit into indentations on the inner wall of a casing of a drug delivery device (for example casing **1259** as illustrated in FIG. 12B). Optionally, anchors **1157** inhibit movement of element **1130** with respect to the drug delivery device. Optionally other means may be used to stabilize element **1130** with respect to a delivery device, for example glue and/or other sorts of anchors and/or fittings.

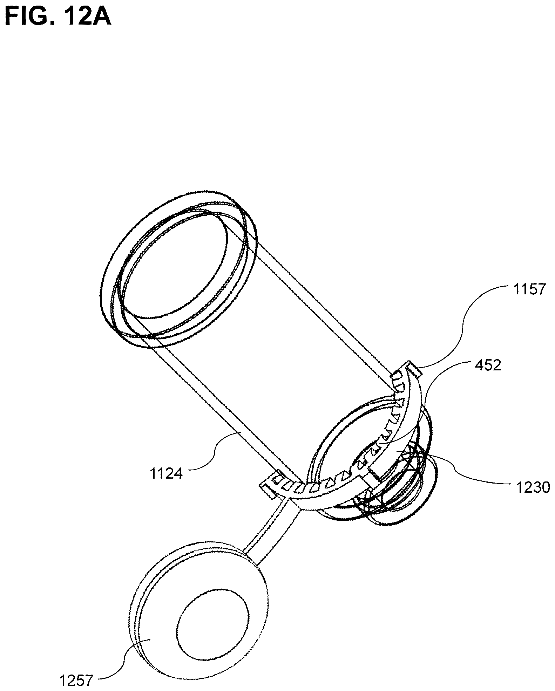

FIG. 12A is a perspective view illustration of a drug reservoir with an alternative external anti-rotational friction element **1230** in accordance with an embodiment of the present invention. Optionally element **1230** only partially surrounds reservoir **1124.** Element **1230** is optionally molded in one step with other elements of a drug delivery device. For example, element **1230** includes an elastomeric button cover **1257** for the delivery device.

FIG. 12B illustrates an internal view of friction element **1230** installed into a top half of a casing **1259** of a drug delivery device. Element **1230** optionally serves to inhibit rotation of reservoir **1124.** Optionally cover **1257** seals a hole in casing **1259** providing a water tight seal. The hole optionally provides access for a user to push an activation button of the device.

Optionally an outer friction element may include various anisotropic friction elements as described herein above for example a sprag and/or an anisotropically bending beam and/or a clutch dog and/or a virtual element and/or an anisotropic friction surface. Friction to inhibit rotation of the reservoir may optionally be provided by multiple elements.

### Exemplary connecting of a driver to a drug cartridge

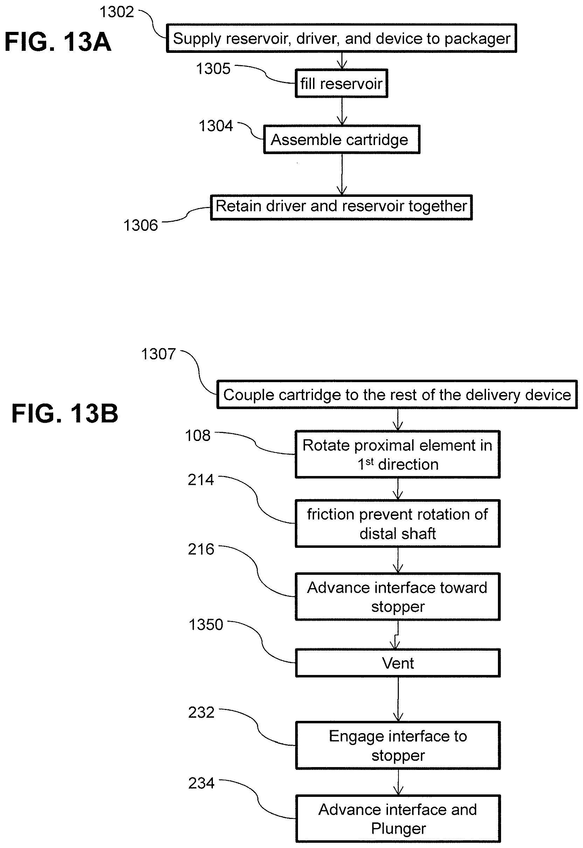

FIG. 13A is a flow chart illustration of a method of retaining a plunger driver to a reservoir in accordance with an embodiment of the current invention. In some embodiments, a plunger driver of a drug delivery cartridge is held to a drug reservoir by a friction element. For example, friction between the friction element and an inner wall of the reservoir may inhibit separation of the driver from the reservoir. Optionally, the friction element includes an anti-rotation friction disk. For example, the friction element may retain the cartridge in an assembled condition prior to insertion of the cartridge into a delivery device.

In some embodiments, a drug delivery device is supplied **1302** to a drug packager in a preassembled state. For example, the packager may fill **1305** the reservoir and then perform final assembly of the device. Optionally filling **1305** is performed under high level aseptic conditions. In some embodiments, final assembly is performed under less strict sterility conditions. In some embodiments, it is advantageous to keep the assembly process simple and/or avoid disruption of sterile parts during the final assembly.

In some embodiments, filling **1305** a reservoir may include loading a drug into the reservoir, stoppering the reservoir and/or capping. Optionally filling **1305** is performed by the packager and/or in a highly aseptic automatic filling machine. For example, the reservoir may be loaded with a measured quantity of a fluid. Optionally the fluid will occupy a portion of the reservoir. For example, a liquid payload may be loaded into a distal portion of the reservoir. For example, stoppering may include inserting the plunger seal (which may sometimes be referred to as a stopper and/or as a plunger) into the reservoir. For example, the plunger seal may be inserted into a proximal opening of the reservoir. Optionally the plunger seal will be inserted until it reaches to the proximal end or close to the proximal end of the payload in the reservoir. Stoppering is optionally performed under vacuum. Optionally stoppering includes protecting a sterile needle. For example, the needle may be covered with a needle cap.

In some embodiments, the reservoir is assembled **1304** to a drug cartridge. For example, after filling **1305,** the packager may assembly **1304** the cartridge. For example, assembly **1304** may include inserting distal portion of plunger driver into a proximal end of a reservoir. In some embodiments, the distal portion of the driver may include a friction element. Optionally, the plunger driver is inserted during assembly until it engages the plunger seal. Alternatively or additionally, during assembly **1304** of the cartridge, contact with the plunger seal may be avoided. For example, during assembly, the distal end of the driver may not reach and/or may not contact and/or may not fully engage the plunger seal. In some embodiments, by avoiding contact between the driver and the plunger seal, the packager may avoid complicated fitting and/or disruption of the sterile portion of the plunger seal and/or disruption of the payload (for example pressure from the driver may disrupt payload and/or causing a leak of the payload). For example, contact may be avoided during assembly of the device and/or subsequent handling, storage and/or transport of the device. Optionally the friction element may retain **1306** the driver and the reservoir together, for example, during handling of the cartridge and/or during assembly of the device.

FIG. 13B is a flow chart illustration of assembly and/or use of a drug delivery device and/or a drug cartridge in accordance with an embodiment of the current invention. Optionally, a plunger seal remain disengaged from a plunger driver during a portion of the assembly process. For example, while the plunger driver is being connected to the cartridge, it may remain disengaged from the plunger seal. Optionally, after assembly of the cartridge, the cartridge may be coupled to the rest of a drug delivery device. Optionally, after coupling the cartridge to the rest of the device, the plunger driver may engage the plunger seal and/or discharge the drug. Alternatively or additionally, the plunger driver may engage the plunger seal before fully assembling the device.

In some embodiments, assembly of a drug delivery device includes coupling 1307 a cartridge to the rest of the device. For example, in a preloaded device, a cartridge may be coupled 1307 to the rest of the device before delivery to a user. For example, preloading including assembly and/or coupling of the cartridge to the device may be performed by a drug packager. Alternatively or additionally, the cartridge may be coupled to the device by a supplier (for example a pharmacist and/or a medical professional). Alternatively or additionally, a device may be delivered to a user in a not fully assembled state. For example, the user may couple 1307 the cartridge to the rest of the device. For example, coupling 1307 a cartridge may include connecting a plunger driver to an actuator (for example a motor). For example, the motor may be connected to the driver via a transmission. For example, coupling 1307 a cartridge may include immobilizing a portion of the cartridge (for example a reservoir) with respect to the rest of the device.

In some embodiments, activating the device will cause the plunger driver to engage the plunger seal and/or will cause the plunger driver to drive the plunger seal into the reservoir to discharge the drug. For example, activating the device may cause the plunger driver to drive a plunger seal interface and/or a friction element towards the plunger seal. Optionally, after the plunger seal interface and/or friction element contacts the plunger seal, the driver may drive the plunger seal into the reservoir. Optionally, driving the plunger seal into the reservoir discharges a drug.

In some embodiments, while the plunger driver is advancing towards the plunger seal, the region between the plunger driver and the plunger seal will be vented **1350.** For example, gas intervening between the driver and the plunger seal may be vented **1350** out a vent in the driver and/or an anti-rotation device. For example, the gas may be wanted out the proximal opening of the reservoir. Venting **1350** may facilitate the driver reaching and/or engaging **232** the plunger seal.

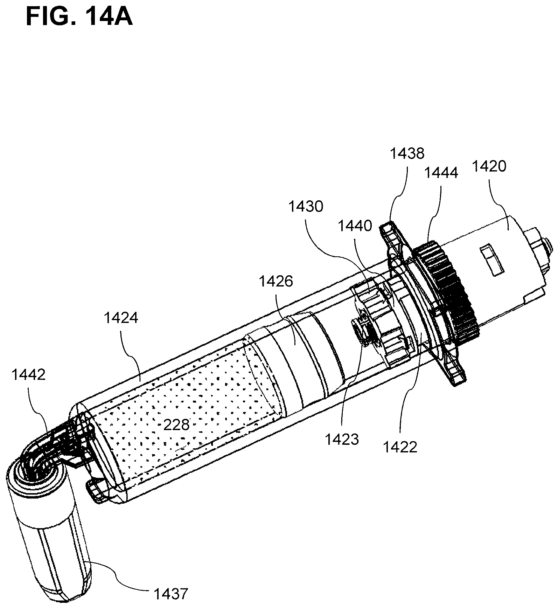

FIG. 14A illustrates a medicine cartridge in accordance with an embodiment of the current invention. The cartridge is illustrated in an exemplary assembled state. In some embodiments, a cartridge includes a plunger driver assembly, a reservoir **1424** and a plunger seal **1426.** Optionally the cartridge is preloaded and/or sterile and/or sealed.

In some embodiments, an assembled cartridge may include a plunger driver assembly connected to a cartridge. Optionally the plunger driver assembly may be partially and/or fully inserted into reservoir **1424.** For example, a plunger driver assembly may include a proximal element **1420.** Optionally proximal element **1420** remains outside and/or proximal to reservoir **1424.** Optionally, a distal portion of the plunger driver assembly is inserted into reservoir **1424,** for example through a proximal opening thereof. For example, the distal portion of the driver assembly may include a friction element **1430.** For example, friction between element **1430** and reservoir **1424** may retain the driver assembly connected to reservoir **1424.** For example, element **1430** is in contact with an inner wall of reservoir **1424.**

In some embodiments, a cartridge is prefilled. For example, a drug **228** may be loaded into reservoir **1424.** Optionally, reservoir **1424** may be sealed with a plunger seal **1426.** Alternatively or additionally, a cartridge may be loaded with a placebo and/or a marker and/or a dye and/or a biological active material.

In some embodiments, friction element **1430** is connected to proximal element **1420** via a base **1440** and/or a pushing shaft **1422** and/or a telescoping shaft **1423.** For example, telescoping shaft **1423** may be connected and/or rotationally fixed to proximal element **1420** such that rotating proximal element **1420** causes rotation of telescoping shaft **1423.** For example telescoping shaft **1423** may be threadably connected (for example via a screw thread) to pushing shaft **1422** such that rotating telescoping shaft **1423** with respect to pushing shaft **1422** causes the driver assembly to telescope (for example extend and/or contract).

In some embodiments, a driver is inserted into a cartridge in a disengaged state. For example, in FIG. 14A, the driver is not engaged to a plunger seal **1426.** Optionally, the cartridge may be stored, shipped and/or coupled to the rest of the drug discharge device while the driver assembly remains disengaged from plunger seal **1426.** Alternatively or additionally, the driver assembly may be engaged to plunger seal **1426** prior to storage, shipping and/or coupling to the rest of the drug discharge device.

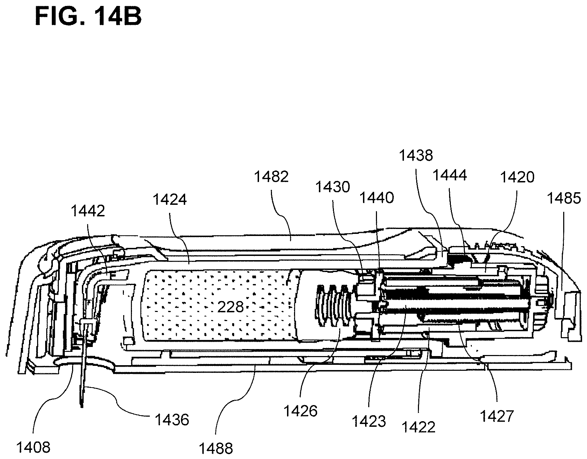

In some embodiments, a cartridge may include a bent needle mount **1442.** For example, needle mount **1442** supports a fluid outlet (for example a hypodermic needle **1436** for example as illustrated in FIG. 14B) at an angle to the longitudinal axis of reservoir **1424.** For example, a cartridge with bent mount **1442** may be useful for a patch injector (for example as illustrated in FIG. 14B). Alternatively or additionally, a cartridge may include a straight needle mount (for example as illustrated in FIGs. 4A, 4B and/or 10A). Alternatively or additionally, a cartridge outlet may include a hub for attachment to an external fluid path and/or a septum. Alternatively or additionally mount **1442** may include a mounting surface for a sterile needle cap **1437.**

In some embodiments, an indicator may be supplied to a drug packager with a drug discharge device. For example, reservoir **1424** and/or the plunger driver assembly (for example including friction pad **1430** and/or pushing shaft **1422** and/or base **1440)** may include a user indicator. Alternatively or additionally, a part supplied by the drug packager (for example plunger seal **1426** and/or a sticker placed on reservoir **1424)** in include a user indicator. For example, when the indicator is indicating some function of the device that is not specific to the drug being packaged, the indicator may be supplied by the device manufacturer. For example, when the indicator is indicating some aspect of treatment that is specific to the drug being packaged, the indicator may be supplied by the device manufacturer.

FIG 14B illustrates a cartridge coupled to a delivery device in accordance with an embodiment of the current invention. In some embodiments, a plunger driver may include a telescoping screw assembly (TSA). For example pushing shaft **1422** may be threadably connected to telescoping shaft **1423,** which is optionally threadably connected to a mid shaft **1427.** Optionally, when shaft **1423** is rotated with respect to pushing shaft **1422** the assembly telescopes.

In some embodiments, when the cartridge is couple to the rest of the injector, reservoir **1424** is immobilized with respect to a housing **1482.** For example flange **1438** may be held to the housing **1482.** For example, flange **1438** and/or reservoir **1424** may include protrusions that snap to housing **1482** and/or a base **1488** thereof. Alternatively or additionally, flange **1438** and/or reservoir **1424** may include an orientation element (for example a flat side) that fits to a matching orientation element on the housing inhibiting rotation of reservoir **1424** with respect to housing **1482.**

In some embodiments, an actuator (for example a motor) is coupled to a transmission element (for example gear **1444).** Optionally the actuator rotates gear **1444** and/or proximal element **1420,** which optionally rotate telescoping shaft **1423** with respect to housing **1482** and/or reservoir **1424.** Optionally, friction between friction element **1430** and an inner wall of reservoir **1424** inhibits rotation of friction element **1430** with respect to reservoir **1424.** Optionally pushing shaft **1422** is connected to friction element **1430** for example via base **1440.** For example, the connection inhibits rotation of shaft **1422** with respect to reservoir **1424.** For example, rotating telescoping shaft **1423** in an expanding direction with respect to reservoir **1424** while immobilizing pushing shaft **1422** to rotation with respect to reservoir optionally causes the telescoping assembly to expand longitudinally. Optionally a hub **1485** in housing **1482** blocks proximal movement of telescoping shaft **1423.** In some embodiments, it is easier to move friction element **1430** and/or plunger seal **1426** linearly in the distal direction than to rotate friction element **1430** with respect to reservoir **1424.** For example, the pitch of the screw thread in the TSA may be small enough that a large rotation is associated with a small linear movement giving a mechanical advantage to linear movement over rotation of friction pad **1430.** Expanding the TSA optionally forces pushing shaft **1422** and/or base **1440** and/or friction element **1430** to move distally.

In some embodiments, a drug discharge device may be supplied with the cartridge coupled thereto and/or with the plunger driver disengaged from plunger seal **1426.** Optionally a user places base **1488** onto his skin and/or inserts needle **1436** through an aperture **1408** in base **1488** into his skin. Optionally, base **1488** includes an adhesive to stick the device to the skin. The user optionally activates an actuator to drive pushing shaft and/or base **1440** and/or friction element **1430** distally until they engage plunger seal **1426.** Optionally, engagement includes a distal surface of friction pad **1430** contacting a proximal surface of plunger seal **1426.** Optionally, further rotation of the actuator after engagement drives plunger seal **1426** distally into reservoir **1424** and/or expels drug **228** out needle **1436.**

### Snap connectors for a friction element

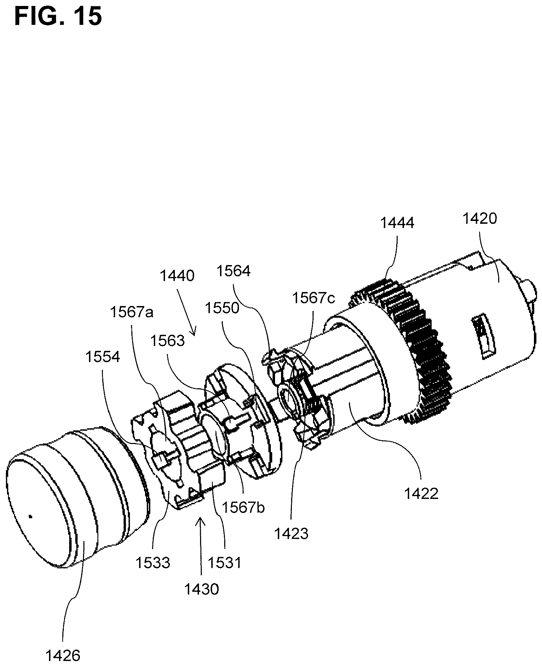

FIG. 15 illustrates a connection between a friction pad and a plunger driver in accordance with an embodiment of the current invention. In some embodiments, a friction element is connected to a plunger driver by a snap connection. Optionally, a vent is provided, allowing air to escape as the friction element moves along a reservoir.

In some embodiments, a friction element may include a snap connection and/or an interference element. For example, a fastener may connect any two components selected from a base, a plunger driver, a friction element and/or a plunger seal. The connector may include a quick connector, a snap, an indentation fitting a protrusion, a barb, an elastic element. A connection may optionally be designed for removal by a user and/or for a permanent connection. Optionally a connection may inhibit movement in one direction and/or in multiple directions.

In some embodiments, a friction pad will be mounted to a plunger driver by one or more connectors. For example, friction pad **1430** includes a channel **1554,** which fits a barbed protrusion **1563.** For example, when pad **1430** is fit over base **1440,** protrusion **1563** snaps into channel **1554** such that pulling pad **1430** off from base **1440** requires much more force than connecting them together, for example more than 10 times as much force and/or more than 100 times and/or more than 1000 times. Optionally, the connection may be permanent. For example, pulling friction pad **1430** off from base **1440** may require enough force to rip friction pad **1430.**

In some embodiments, a friction pad will be mounted to a plunger driver by one or more snap connectors. For example, pushing shaft **1422** includes one or more flexible arm interference elements **1564,** which snap into openings **1550** in base **1563.** A barb or hook in element **1564** optionally catches a side of opening **1550** inhibiting pulling base **1440** away from shaft **1422.** In some embodiments, pulling base **1440** away from shaft **1422** may require much more force than connecting them together for example more than 10 times as much force and/or more than 100 times and/or more than 1000 times. Optionally, the connection may be permanent. For example, pulling base **1440** off from shaft **1422** may require enough force to break interference element **1564.**

In some embodiments, a friction element may be connected to a plunger driver to resist movement in any or all directions. For example, friction pad **1430** is snapped to shaft **1422** (via base **1440)** to resist movement with respect to shaft **1422** in all directions with a force much greater than the force needed to connect the parts. In some embodiments, this may be useful when assembling the driver to a reservoir. For example, friction between pad **1430** and the inner walls of reservoir **1424** keeps pad **1430** connected to reservoir **1424.** Optionally, resistance to axial separation between pad **1430** and the plunger driver keeps the plunger driver connected to reservoir **1424,** for example while assembling the cartridge and/or the delivery device.

In some embodiments a friction pad, a base and/or a driver may be designed to vent air. For example, venting may occur as the pad is inserted into a reservoir. For example, air trapped between the friction pad and a plunger seal may be vented out the proximal end of the reservoir and/or into the space between the friction pad and the proximal end of the reservoir. For example, there may be space between the friction pad and the inner wall of the reservoir that allows air to vent. For example, arms and/or sprags **1533** may contact the inner wall of reservoir **1424.** Optionally ventilation space may be provided between sprags **1533.** Optionally, openings **1550** in base **1440** are aligned with the spaces between sprags **1533** such that air that passes through the spaces between the sprags can further pass through openings **1550.** Alternatively or additionally, a gas vent may include one or more holes that are optionally aligned. For example, a center hole **1567a** in pad **1430** is aligned to a center hole **1567b** in base **1440** which is aligned with a central hole **1567c** in telescoping screw **1423.**

In some embodiments, a friction surface may be adjusted to achieve a desired frictional force. For example in some embodiments a surface of contact between a plunger seal and a reservoir and/or a friction pad and a reservoir may be treated to increase and/or decrease friction. In some embodiments a contact surface between a friction pad and a plunger seal may be treated to increase and/or decrease friction. Optionally the treatment of one friction surface may differ from the treatment of another surface. For example, a surface of contact **1531** between friction pad **1430** and reservoir **1424** may be coated for example with PTFE for example to reduce friction while a surface **1533** of contact between friction pad **1430** and plunger seal **1426** may be coated with a high friction coating (for example a rough coating) and/or textured (for example with ridges) to increase friction. For example, a cylindrical side surface and/or a distal surface of plunger seal **1426** may be coated with PTFE while a proximal surface may include untreated high friction elastomer. A coating of a friction surface may be adjusted according to the material of a reservoir. For example, friction surface **1531** may be coated with PTFE for use with a plastic reservoir and/or coated with another material or uncoated for use with a glass reservoir.

In some embodiments, the area of contact of a friction surface may be adjusted to achieve a desired friction. For example, friction pad **1430** includes four sprags, which contact an inner wall of reservoir **1424** over approximately 12% of its circumference. For example, friction pad **430** includes 10 sprags that contact an inner wall of reservoir **424** over approximately 70% of its circumference. For example, a friction pad may contact an inner wall over between 1 to 20% and/or between 20 to 40% and/or between 40 to 80% and/or between 80 to 100% of its circumference. Optionally a surface of contact may be modified to increase friction in one direction. For example a distal face may have ridges for example as illustrated in FIG. 9 that increase friction between pad **930** and a plunger seal in one direction in comparison to another direction.

In some embodiments, an anti-rotational friction pad may produce enough friction by itself to prevent rotation while driving a plunger. In some embodiments, anti rotational friction of the friction pad may not be sufficient to prevent rotation when driving a plunger, but the sum of anti rotational friction between the friction pad and the plunger seal may be sufficient.

In some embodiments, the contact between a driver and/or a friction pad and a plunger seal may be a flat surface. Optionally this will avoid ambiguity in determining the location time and/or position of contact between a driver and a plunger seal.

### Exemplary dimensions of a drug delivery device

In some embodiments the payload of a reservoir (for example a syringe) may include, for example between 0.5 and 2ml and/or between 2 and 4ml and/or between 4 and 5 ml of a drug and/or more. In some embodiments, the injector may discharge the entire payload as a single dose. A drug delivery device may include, for example, a pen injector, and/or an internally powered driver to drive the plunger and/or discharge the payload. For the sake of this application, an internally powered injector driver may be defined as a drive mechanism powered by energy stored at least temporarily within the injector. Power may be stored in a power supply, for instance as chemical potential (for example a chemical that produces an expanding gas and/or a battery) and/or mechanical potential (for example stored in an elastic member and/or a spring and/or torque spring and/or a pressurized gas). For example, the driver may be designed to discharge the payload over a time period ranging between 20 and 120 seconds and/or between 120 and 600 seconds and/or longer. In some embodiments, discharge may be driven by a driver. An internally powered driver may be powered by various mechanisms including for example a motor (including for example a DC motor, an actuator, a brushless motor) and/or a transmission including for example a telescoping assembly and/or a threaded element and/or a gear and/or a coupling and/or an elastic mechanism (for example a spring and/or a rubber band) and/or an expanding gas and/or a hydraulic actuator).

A drug delivery device in accordance with some embodiments of the current invention may include reservoir. For example, a reservoir may include a medicine container and/or a standard type syringe. Optionally a standard type syringe may be preloaded with medicine using standard equipment and/or in an aseptic room. A preloaded standard type syringe may optionally include a proximal opening. A plunger may optionally seal the proximal opening and/or protect the sterility of the contents of the syringe. A sterile needle (for example a hollow needle) may optionally be connected to the syringe barrel. For example, the hollow of the needle may be in fluid communication with the interior of the barrel. The needle may optionally be rigidly attached to the distal end of the barrel. The sterility of all and/or part of the needle may for example be protected by a sterile cover. The sterile cover may remain on the needle when the syringe is supplied and/or installed into an injector. For example, the medicine container may optionally include a cylindrical barrel rigidly attached to a needle. Optionally, the long axes of the needle and barrel of the syringe may be parallel and/or coaxial. Optionally, the needle may be mounted on the distal end of the barrel. Optionally the needle point may be pointing in the distal direction. In some embodiments, a plunger may slide axially along the inside of the barrel to discharge a medicine payload. For example, the medicine may be discharged through the hollow needle.

In some embodiments, the force to insert the needle to the skin of a patient may range for example between 0.02 to 0.2 N and/or between 0.2 and 0.5 N. Optionally, the force required to inject the drug (for example the force on a syringe plunger) may range for example between 5 to 60 N. For example, the force required to inject the drug may depend on the injection rate and/or the viscosity of the drug and/or the syringe geometry and/or the needle dimensions.

For example in the event of an occlusion and/or at the end of delivery, the linear force generated by the device may increase to the level of up to 60 N. A needle safeguarding mechanism (for example a needle retraction mechanism) may be sensitive to the force. For example, mechanism may include a snap that gives way at 40 N returning the needle to the retracted position. In some embodiments, a needle protection mechanism may be triggered by a linear force greater than, for example, between 10 to 60 N.

In some embodiments, the stress to inject a medicine and/or to trigger safeguarding of a needle may include a torque. For example, injection of medicine may be driven by a plunger. The plunger may optionally be driven by a threaded assembly, for example a threaded screw and/or teeth and/or a telescoping assembly. Optionally the pitch of the teeth and/or an associated screw may range for example between 0.5 and 2 mm. The diameter of the screw may range for example between 3 and 15 mm. The torque to power injection may range for example between 0.2 and 1.0 N*cm. The trigger torque (the torque at which the needle safeguarding is triggered) may range for example between to 0.5 to 2 and/or from 2 to 4 and/or from 4 to 10N*cm.

During injection, the linear movement of a plunger may range for example between 10-50mm. The length of movement of the plunger may vary for example with the volume of medicine to be injected that may range for example between 0.5 to 3ml.

In some embodiments, discharge may be driven by a torque. For example, the driver may apply torque to threaded element pushing a plunger. In some embodiments, a time of discharge may depend on the fill volume and/or viscosity For example the expected injection speeds may be Injection speed depend on viscosity, for example for viscosity ranging from 1cp to 15 cp the expected injection rage may range between 30 to 40sec/1ml, for example for viscosity ranging from 15cp to 60 cp the expected injection rate may range between 35 to 60 sec/ml for viscosity above 60 cp the expected injection rate may range between 53 to 67sec/1ml. The maximum and/or minimum expected injection time may for example be the maximum and/or minimum allowed fill volume divided by an injection rate. For example an expected time of discharge may range for example between 24 to 48 seconds (for example for between 0.8 and 1.2ml of fluid having a viscosity ranging between 1 to 15cp) and/or between 36 to 68 seconds (for example for between 1.2 and 1.7 ml of fluid having a viscosity ranging between 1 to 15cp) and/or between 51 to 92 seconds (for example for between 1.7 and 2.3ml of fluid having a viscosity between 1 to 15cp) and/or between 70 to 150 seconds (for example for 2.0 to 2.5 ml of fluid having a viscosity of between 15 and 40cp) and/or between 120 seconds and 3 minutes for larger volumes and/or viscosities.

In some embodiments, the drug delivery device may be configured to operate independently and/or be handheld. For example, the device may have a weight ranging between 10 grams to 30 grams and/or between 30 grams to 150 grams and/or between 150 grams to 500 grams. Optionally the drug may be contained within the device. Optionally the fluid path of the drug from the reservoir to the injection needle may be within the device. Optionally the power supply may be within the device. Optionally the device may be operable with one hand.

It is expected that during the life of a patent maturing from this application many relevant technologies will be developed and the scope of the terms are intended to include all such new technologies a priori.

As used herein the term "about" refers to ± 5%.

The terms "comprises", "comprising", "includes", "including", "having" and their conjugates mean "including but not limited to".

The term "consisting of' means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Citation or identification of any reference in this application shall not be construed as an admission that such reference is available as prior art to the present invention. To the extent that section headings are used, they should not be construed as necessarily limiting.

It will be appreciated by those skilled in the art that changes could be made to the embodiments described above without departing from the inventive concept thereof. It is understood, therefore, that this invention is not limited to the particular embodiments disclosed, but it is intended to cover modifications within the scope of the present invention as defined by the appended claims.

## Claims

1. An assembly for driving a plunger (326) in a drug reservoir (324; 424; 1124;1424), the plunger (326) dividing between a drug in a distal portion of the reservoir (324; 424; 1124; 1424) and a proximal opening of the reservoir (324; 424; 1124; 1424); the assembly comprising:
a telescoping shaft (1423) that extends by rotation of a driving element in a first rotational direction with respect to a distal shaft (322);
a distal end of said distal shaft (322) configured to engage the plunger (326);
and
a friction element (430, 1430) extending from said distal shaft (322);
**characterized in that**
the friction element (430, 1430) comprises a flexible disk and a plurality of independent flexible radial elements (352, 452) extending radially from the flexible disk to contact an interior side wall of the reservoir (324; 424; 1124; 1424), said flexible radial elements (352, 452) extending from the disk tilting off toward the first rotational direction to wedge between said interior wall and said distal shaft (322) when said distal shaft (322) is rotated in said first rotational direction, said radial elements (352, 452) having respective leading edges (456a) and trailing edges (456b), at least one of the leading edges (456a) extending radially from the disk at a first angle and a trailing edge (456b) associated with a same radial element (353, 452) as the at least one of the leading edges (456a) extending radially from the disk at a second angle, the first angle being different than the second angle.

2. The assembly of claim 1, wherein each radial element (352, 452) is configured to function as a sprag (353) having a long axis and wherein said long axis intersects said interior side wall.

3. The assembly of claim 1, further comprising:
a plunger interface (332) configured to engage the plunger (326); said plunger interface (332) irrotationally interconnecting said distal shaft (322) and the plunger (326) when said plunger interface (322) is engaged to the plunger (326).

4. The assembly of claim 1, further comprising:
a vent (450) for releasing gas from a space between the friction element (430, 1430) and the plunger (326).

5. The assembly of claim 1, wherein said friction element (430, 1430) is located proximal to said distal end of said distal shaft (322).

6. The assembly of claim 1, wherein said wedging is longitudinal such that moving said distal shaft (322) distally with respect to said reservoir (324; 424; 1124; 1424) reduces a normal force between said friction element (430, 1430) and said interior wall.

7. The assembly of claim 1, wherein a resistance to movement of said distal shaft (322) in said first rotational direction due to friction between said friction element (430, 1430) and said interior wall is higher than a resistance to movement of said distal shaft (322) in a direction opposite said first rotational direction due to friction between said friction element (430), 1430) and said interior wall.

8. The assembly of claim 1, wherein said friction element (430, 1430) is a first friction element (430, 1430), and further comprising:
a second friction element extending from a drug delivery device to contact an exterior side wall of the reservoir (324; 424; 1124; 1424) when said reservoir (324; 424; 1124; 1424) is inserted into said drug delivery device, said second friction element is configured and oriented to wedge between said exterior wall and said reservoir (324; 424; 1124; 1424) when said reservoir (324; 424; 1124; 1424) is rotated in a second rotational direction.

9. The assembly of claim 8, wherein said second rotational direction is opposite said first rotational direction.

10. A method of advancing a plunger (326) in a drug reservoir (324; 424; 1124; 1424) comprising:
providing a telescoping assembly that extends by rotating a driver in a first rotational direction with respect to a pushing shaft (422) mated to the driver, said pushing shaft (422) including a friction element (430, 1430) in contact with a wall of the reservoir (324; 424; 1124; 1424), said friction element (430, 1430) comprising a flexible disk and a plurality of independent flexible radial elements (352, 452) extending radially from the flexible disk, said flexible radial elements (352, 452) having respective leading edges (456a) and trailing edges (456b), at least one of the leading edges (456a) extending radially from the disk at a first angle and a trailing edge (456b) associated with a same radial element (352, 452) as the at least one of the leading edges (456a) extending radially from the disk at a second angle, the first angle being different than the second angle;
rotating said driver in said first rotational direction;
wedging said friction element (430,1430) between said pushing shaft (422) and said wall as result of said rotating;
inhibiting rotation of said pushing shaft (422) as a result of said wedging; and
unwedging said friction element (430, 1430) to facilitate advancing of said pushing shaft (422).

11. The method of claim 10 , wherein said wedging is substantially the only process impeding rotation of said pushing shaft (422) with respect to the reservoir (324; 424; 1124; 1424).

12. The method of claim 10, wherein said wedging includes increasing a normal force.

13. The method of claim 10 , further comprising:
reducing a normal force between said friction element (430, 1430) and said wall of the reservoir (324; 424; 1124; 1424) when said pushing shaft (422) moves in a distal direction.

14. The method of claim 10, wherein the drug reservoir (324; 424; 1124; 1424) includes a plunger (326), the method further comprising:
reducing a normal force between said friction element (430, 1430) and said wall of the reservoir (432) when said pushing shaft (422) pushes distally against said plunger (326).

15. A drug delivery device comprising:
a housing (1482) including a channel;
a drug reservoir (324; 424; 1124; 1424) fitting into said channel;
the assembly of claim 1, and
an external friction element (1130) extending from the housing (1428) to said channel to contact an exterior side wall of the reservoir (324; 424; 1124; 1424) when said reservoir (324; 424; 1124; 1424) is inserted into said channel, said external friction element (1130) configured and oriented to wedge between said exterior wall and said reservoir (324; 424; 1124; 1424) when said reservoir (324; 424; 1124; 1424) is rotated in a second rotational direction.

16. The assembly of claim 15 , wherein said channel is cylindrical and includes an opening at one end and wherein said reservoir (324; 424; 1124; 1424) slides longitudinally through said opening into said channel and wherein said external friction element (1130) has increased friction resistance to movement in said second rotational direction than to movement in said longitudinal direction.

17. The assembly of claim 4, wherein said vent (450) is formed in at least one component selected from the group consisting of said friction element (430, 1430), between said friction element (430, 1430) and said side wall of said reservoir (324; 424; 1124; 1424), said distal shaft (322), and between said distal shaft (322) and said friction element (430, 1430).

18. The assembly of claim 17 , including at least two vents (450) in at least two components and wherein said at least two vents (450) are aligned such that gas passing through a first vent (450) in a first of said two components is channeled to a second vent (450) in a second of said components.

## Patentansprüche

1. Anordnung zum Treiben eines Kolbens (326) in einem Arzneimittelreservoir (324; 424; 1124; 1424), wobei der Kolben (326) ein Arzneimittel in einem distalen Bereich des Reservoirs (324; 424; 1124; 1424) und eine proximale Öffnung des Reservoirs (324; 424; 1124; 1424) voneinander trennt; wobei die Anordnung umfasst:
eine Teleskopwelle (1423), die durch die Drehung eines Antriebselements in einer ersten Drehrichtung in Bezug auf eine distale Welle (322) ausfährt;
ein distales Ende der distalen Welle (322), das konfiguriert ist für den Eingriff mit dem Kolben (326); und
ein Reibelement (430, 1430), das sich von der distalen Welle (322) erstreckt;
**dadurch gekennzeichnet, dass**
das Reibelement (430, 1430) eine flexible Scheibe und eine Mehrzahl von unabhängigen flexiblen radialen Elementen (352, 452) umfasst, die sich für den Kontakt mit einer Innenseitenwand des Behälters (324; 424; 1124; 1424) radial von der flexiblen Scheibe erstrecken, wobei sich die von der Scheibe erstreckenden flexiblen radialen Elemente in die erste Drehrichtung neigen, um sich zwischen der Innenwand und der distalen Welle (322) zu verkeilen, wenn die distale Welle (322) in der ersten Drehrichtung gedreht wird, wobei die radialen Elemente (352, 452) jeweilige Vorderkanten (456a) und Hinterkanten (456b) aufweisen, wobei sich zumindest eine der Vorderkanten (456a) unter einem ersten Winkel radial von der Scheibe erstreckt und eine Hinterkante (456b), die demselben radialen Element (353, 452) zugeordnet ist wie die wenigstens eine Vorderkante (456a), sich unter einem zweiten Winkel radial von der Scheibe erstreckt, wobei sich der erste Winkel von dem zweiten Winkel unterscheidet.

2. Anordnung nach Anspruch 1, wobei jedes radiale Element (352, 452) derart konfiguriert ist, dass dieses als Hemmschuh (353) wirkt, der eine Längsachse hat, die die Innenseitenwand schneidet.

3. Anordnung nach Anspruch 1, ferner umfassend:
ein Kolbenzwischenstück (332), das für den Eingriff mit dem Kolben (326) konfiguriert ist; wobei das Kolbenzwischenstück (322) die distale Welle (322) und den Kolben (326) rotationsfrei verbindet, wenn sich das Kolbenzwischenstück (322) mit dem Kolben (326) in Eingriff befindet.

4. Anordnung nach Anspruch 1, ferner umfassend:
eine Lüftungsöffnung (450) zur Freisetzung von Gas aus einem Raum zwischen dem Reibelement (430, 1430) und dem Kolben (326).

5. Anordnung nach Anspruch 1, wobei sich das Reibelement (430, 1430) in der Nähe des distalen Endes der distalen Welle (322) befindet.

6. Anordnung nach Anspruch 1, wobei das Verkeilen längs erfolgt, so dass eine bezüglich des Reservoirs (324; 424; 1124; 1424) distale Bewegung der distalen Welle (322) eine Normalkraft zwischen dem Reibelement (430, 1430) und der Innenwand verringert.

7. Anordnung nach Anspruch 1, wobei ein durch die Reibung zwischen dem Reibelement (430, 1430) und der Innenwand bedingter Widerstand gegen eine Bewegung der distalen Welle (322) in der ersten Drehrichtung höher ist als ein durch die Reibung zwischen dem Reibelement (430, 1430) und der Innenwand bedingter Widerstand gegen eine Bewegung der distalen Welle (322) in eine zu der ersten Drehrichtung entgegengesetzte Richtung.

8. Anordnung nach Anspruch 1, wobei das Reibelement (430, 1430) ein erstes Reibelement (430, 1430) ist und wobei die Anordnung ferner umfasst:
ein zweites Reibelement, das sich von einer Arzneimittelabgabevorrichtung erstreckt und an einer Außenseitenwand des Reservoirs (324; 424; 1124; 1424) anliegt, wenn das Reservoir (324; 424; 1124; 1424) in die Arzneimittelabgabevorrichtung eingesetzt ist, wobei das Reibelement derart konfiguriert und orientiert ist, dass dieses sich zwischen der Außenwand und dem Reservoir (324; 424; 1124; 1424) verkeilt, wenn das Reservoir (324; 424; 1124; 1424) in eine zweite Richtung gedreht wird.

9. Anordnung nach Anspruch 8, wobei die zweite Drehrichtung zur ersten Drehrichtung entgegengesetzt ist.

10. Verfahren zum Vortreiben eines Kolbens (326) in einem Arzneimittelreservoir (324; 424; 1124; 1424), umfassend:
das Bereitstellen einer Teleskopanordnung, die ausfährt, indem ein Treiber mit Bezug auf eine an den Treiber angepasste Schiebewelle (422) in einer ersten Drehrichtung gedreht wird, wobei die Schiebewelle (422) ein Reibelement (430, 1430) umfasst, das sich mit einer Wand des Reservoirs (324; 424; 1124; 1424) in Kontakt befindet, wobei das Reibelement (430,1430) eine flexible Scheibe und eine Mehrzahl von unabhängigen flexiblen radialen Elementen (352, 452) umfasst, die sich von der flexiblen Scheibe radial erstrecken, wobei die flexiblen radialen Elemente (352, 452) jeweilige Vorderkanten (456a) und Hinterkanten (456b) aufweisen, wobei sich zumindest eine der Vorderkanten (456a) unter einem ersten Winkel radial von der Scheibe erstreckt und eine Hinterkante (456b), die demselben radialen Element (352, 452) zugeordnet ist wie die wenigstens eine der Vorderkanten (456a), sich unter einem zweiten Winkel radial von der Scheibe erstreckt, wobei sich der zweite Winkel von dem ersten Winkel unterscheidet;
das Drehen des Treibers in die erste Drehrichtung;
das Verkeilen des Reibelements (430, 1430) zwischen der Schiebewelle (422) und der Wand infolge der Drehung;
das Hemmen der Drehung der Schiebewelle (422) infolge der Verkeilung; und
das Aufheben der Verkeilung des Reibelements (430, 1430) um einen Vorschub der Schiebewelle (422) zu ermöglichen.

11. Verfahren nach Anspruch 10, wobei das Verkeilen im Wesentlichen der einzige Vorgang ist, der eine Drehung der Schiebewelle (422) in Bezug auf das Reservoir (324; 424; 1124; 1424) hemmt.

12. Verfahren nach Anspruch 10, wobei das Verkeilen das Vergrößern der Normalkraft beinhaltet.

13. Verfahren nach Anspruch 10, ferner umfassend:
das Verringern einer Normalkraft zwischen dem Reibelement (430, 1430) und der Wand des Reservoirs (324; 424; 1124; 1424), wenn sich die Schiebewelle (422) in eine distale Richtung bewegt.

14. Verfahren nach Anspruch 10, wobei das Arzneimittelreservoir (324; 424; 1124; 1424) einen Kolben (326) enthält, wobei das Verfahren ferner umfasst:
das Verringern einer Normalkraft zwischen dem Reibelement (430, 1430) und der Wand des Reservoirs (432), wenn die Schiebewelle (422) distal gegen den Kolben (326) drückt.

15. Arzneimittelabgabevorrichtung, umfassend:
ein Gehäuse (1482) mit einem Kanal;
ein Arzneimittelreservoir (324; 424; 1124; 1424), das in den Kanal eingepasst ist;
die Anordnung gemäß Anspruch 1 und
ein externes Reibelement (1130), das sich von dem Gehäuse (1428) zu dem Kanal erstreckt zur Anlage an einer Außenseitenwand des Reservoirs (324; 424; 1124; 1424), wenn das Reservoir (324; 424; 1124; 1424) in den Kanal eingesetzt ist, wobei das externe Reibelement (1130) für ein Verkeilen zwischen der Außenwand und dem Reservoir (324; 424; 1124; 1424) konfiguriert und orientiert ist, wenn das Reservoir (324; 424; 1124; 1424) in einer zweiten Drehrichtung gedreht wird.

16. Anordnung nach Anspruch 15, wobei der Kanal zylindrisch ist und an einem Ende eine Öffnung hat und wobei das Reservoir (324; 424; 1124; 1424) in Längsrichtung durch die Öffnung in den Kanal gleitet und wobei das externe Reibelement (1130) einen höheren Reibwiderstand gegen eine Bewegung in der zweiten Drehrichtung als gegen eine Bewegung in der Längsrichtung aufweist.

17. Anordnung nach Anspruch 4, wobei die Lüftungsöffnung (450) in wenigstens einer Komponente gebildet ist, die aus der Gruppe ausgewählt ist, die aus dem Reibungselement (430, 1430), zwischen dem Reibungselement (430, 1430) und der Seitenwand des Reservoirs (324; 424; 1124; 1424), der distalen Welle (322) und zwischen der distalen Welle (322) und dem Reibungselement (430, 1430) besteht.

18. Anordnung nach Anspruch 17, enthaltend zumindest zwei Lüftungsöffnungen (450) in zumindest zwei Komponenten und wobei die beiden Lüftungsöffnungen (450) derart ausgerichtet sind, dass Gas, das durch eine erste Lüftungsöffnung (450) in einer ersten der beiden Komponenten zu einer zweiten Lüftungsöffnung (450) in einer zweiten der Komponenten gelenkt wird.

## Revendications

1. Dispositif pour entraîner un piston (326) dans un réservoir de médicament (324; 424; 1124; 1424), le piston (326) séparant un médicament dans une partie distale du réservoir (324; 424; 1124; 1424) et une ouverture proximale du réservoir (324; 424; 1124; 1424); le dispositif comprenant:
un arbre télescopique (1423) qui s'étend dans un premier sens de rotation par rapport à un arbre distal (322) par la rotation d'un élément d'entraînement;
une extrémité distale de l'arbre distal (322) configurée pour s'engager dans le piston (326); et
un élément de friction (430, 1430) s'étendant de l'arbre distal (322);
**caractérisé en ce que**
l'élément de friction (430, 1430) comprend un disque flexible et une pluralité d'éléments radiaux flexibles indépendants (352, 452) s'étendant radialement du disque flexible pour entrer en contact avec une paroi latérale intérieure du réservoir (324; 424; 1124; 1424), lesdits éléments radiaux flexibles s'étendant dudit disque étant inclinés vers ledit premier sens de rotation pour se coincer entre ladite paroi intérieure et ledit arbre distal (322) lorsque ledit arbre distal (322) est tourné dans ledit premier sens de rotation, lesdits éléments radiaux (352, 452) ayant des bords d'attaque (456a) et des bords de fuite (456b) respectifs, dans lequel au moins un desdits bords d'attaque (456a) s'étend radialement dudit disque selon un premier angle et un bord de fuite (456b) associé au même élément radial (353, 452) que ledit au moins un bord d'attaque (456a) s'étend radialement dudit disque selon un second angle, ledit premier angle étant différent dudit second angle.

2. Dispositif selon la revendication 1, dans lequel chaque élément radial (352, 452) est configuré pour agir comme un sabot (353) ayant un axe longitudinal coupant la paroi latérale intérieure.

3. Dispositif selon la revendication 1, comprenant en outre:
une interface de piston (332) configurée pour s'engager avec le piston (326); dans lequel l'interface de piston (332) relie de manière non rotative l'arbre distal (322) et le piston (326) lorsque l'interface de piston (332) est engagé dans le piston (326).

4. Dispositif selon la revendication 1, comprenant en outre:
une ouverture de ventilation (450) pour libérer le gaz d'un espace entre l'élément de friction (430, 1430) et le piston (326).

5. Dispositif selon la revendication 1, dans lequel l'élément de friction (430, 1430) est situé près de l'extrémité distale de l'arbre distal (322).

6. Dispositif selon la revendication 1, dans lequel le coinçage est longitudinal de sorte que le mouvement distal de l'arbre distal (322) par rapport au réservoir (324; 424; 1124; 1424) réduit une force normale entre l'élément de friction (430, 1430) et la paroi intérieure.

7. Dispositif selon la revendication 1, dans lequel une résistance au mouvement de l'arbre distal (322) dans le premier sens de rotation causée par la friction entre l'élément de friction (430, 1430) et la paroi intérieure est plus élevée qu'une résistance au mouvement de l'arbre distal (322) dans un sens opposé au premier sens de rotation causée par la friction entre l'élément de friction (430, 1430) et la paroi intérieure.

8. Dispositif selon la revendication 1, dans lequel l'élément de friction (430, 1430) est un premier élément de friction (430, 1430), et le dispositif comprenant en outre:
un deuxième élément de friction s'étendant d'un dispositif d'administration de médicaments et venant au contact avec une paroi latérale extérieure du réservoir (324; 424; 1124; 1424) lorsque le réservoir (324; 424; 1124) est inséré dans le dispositif d'administration de médicament, l'élément de friction étant configuré et orienté de manière à se coincer entre la paroi extérieure et le réservoir (324; 424; 1124; 1424) lorsque le réservoir (324; 424; 1124; 1424) est tourné dans un deuxième sens.

9. Dispositif selon la revendication 8, dans lequel le deuxième sens de rotation est opposé au premier sens de rotation.

10. Procédé pour faire avancer un piston (326) dans un réservoir de médicament (324; 424; 1124; 1424), comprenant:
fournir un ensemble télescopique qui s'étend en faisant tourner un pousseur dans un premier sens de rotation par rapport à une tige de poussée (422) adapté au pousseur, la tige de poussée (422) comprenant un élément de friction (430, 1430) qui se trouve en contact avec une paroi du réservoir (324; 424; 1124; 1424);
l'élément de friction (430, 1430) comprenant un disque flexible et une pluralité d'éléments radiaux flexibles indépendants (352, 452) s'étendant radialement du disque flexible, les éléments radiaux flexibles (352, 452) ayant des bords d'attaque (456a) et des bords de fuite (456b) respectifs, au moins un desdits bords d'attaque (456a) s'étendant radialement dudit disque selon un premier angle et un bord de fuite (456b) associé au même élément radial (352, 452) que ledit au moins un desdits bords d'attaque (456a) s'étendant radialement dudit disque selon un second angle, ledit second angle étant différent dudit premier angle;
faire tourner le pousseur dans le premier sens de rotation;
coincer l'élément de friction (430, 1430) entre la tige de poussée (422) et la paroi suite à la rotation;
empêcher la rotation de la tige de poussée (422) en raison du coincement;
supprimer le coincement de l'élément de friction (430, 1430) pour permettre l'avancement de la tige de poussée (422).

11. Procédé selon la revendication 10, dans lequel le coincement est sensiblement la seule opération qui empêche la rotation de la tige de poussée (422) par rapport au réservoir (324; 424; 1124; 1424).

12. Procédé selon la revendication 10, dans lequel le coincement comprend l'augmentation de la force normale.

13. Procédé selon la revendication 10, comprenant en outre:
réduire une force normale entre l'élément de friction (430, 1430) et la paroi du réservoir (324; 424; 1124; 1424) lorsque la tige de poussée (422) se déplace dans une direction distale.

14. Procédé selon la revendication 10, dans lequel le réservoir de médicament (324; 424; 1124; 1424) comprend un piston (326), le procédé comprenant en outre:
réduire une force normale entre l'élément de friction (430, 1430) et la paroi du réservoir (432) lorsque la tige de poussée (422) pousse distalement contre le piston (326).

15. Dispositif d'administration de médicaments comprenant :
un boîtier (1482) présentant un canal;
un réservoir de médicaments (324; 424; 1124; 1424) installé dans ledit canal;
le dispositif selon la revendication 1, et
un élément de friction externe (1130) s'étendant du boîtier (1428) au canal pour engager une paroi latérale externe du réservoir (324; 424; 1124; 1424) lorsque le réservoir (324; 424; 1124; 1424) est inséré dans le canal, l'élément de friction externe (1130) étant configuré et orienté pour se coincer entre la paroi extérieure et le réservoir (324; 424; 1124; 1424) lorsque le réservoir (324; 424; 1124; 1424) est tourné dans un deuxième sens de rotation.

16. Dispositif selon la revendication 15, dans lequel le canal est cylindrique et présente une ouverture à une extrémité, et dans lequel le réservoir (324; 424; 1124; 1424) coulisse longitudinalement à travers l'ouverture dans le canal, et dans lequel l'élément de friction externe (1130) présente une résistance de friction plus élevée au mouvement dans le deuxième sens de rotation qu'au mouvement dans le sens longitudinal.

17. Dispositif selon la revendication 4, dans lequel l'ouverture de ventilation (450) est formée dans au moins un composant choisi dans le groupe constitué par l'élément de friction (430, 1430), entre l'élément de friction (430, 1430) et la paroi latérale du réservoir (324; 424; 1124; 1424), l'arbre distale (322) et entre l'arbre distale (322) et l'élément de friction (430, 1430).

18. Dispositif selon la revendication 17, comprenant au moins deux ouvertures de ventilation (450) dans au moins deux composants et dans lequel les deux ouvertures de ventilation (450) sont alignées de telle sorte que le gaz passant à travers une première ouverture de ventilation (450) dans un premier des deux composants est dirigé vers une deuxième ouverture de ventilation (450) dans un deuxième des composants.
